# EUROPÄISCHE PATENTANMELDUNG

(11) **EP 0 873 991 A1**
(43) Veröffentlichungstag der Anmeldung: **28.10.1998**
(21) Anmeldenummer: 98810320.6
(22) Anmeldetag: 15.04.1998
(51) Int. Cl.: C07D 211/46, C08K 5/3435, C07D 211/58

(54) **Polyalkylenglykolgruppen enthaltende gehinderte Amine als Stabilisatoren**

(30) Priorität: 23.04.1997 CH 951/97
(71) Anmelder: Ciba Specialty Chemicals Holding Inc., 4057 Basel (CH)
(72) Erfinder: Steinmann, Alfred, 1724 Praroman (CH)

(57) **Zusammenfassung**

Beschrieben werden Verbindungen der Formel 1 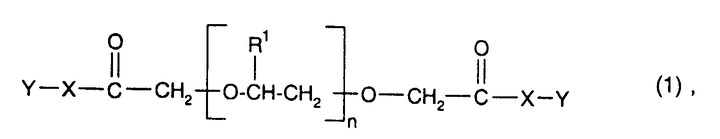 und Verbindungen der Formel 2 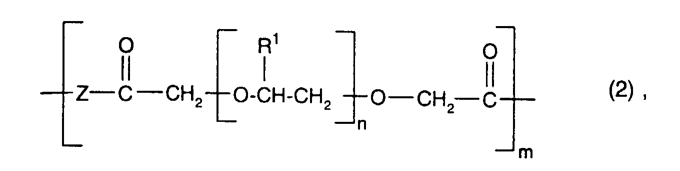 worin X, Y und Z sowie die Reste R¹ als auch m und n die in Anspruch 1 angegebene Bedeutung haben.

Die genannten Verbindungen dienen vorteilhaft z. B. zur Stabilisierung organischen Materials gegen den schädigenden Einfluss von Licht, Sauerstoff und/oder Wärme.

## Beschreibung

Die Erfindung betrifft neue wasserlösliche polymere HALS-Verbindungen, die durch Umsetzung gehinderter Amine [HALS], die funktionelle Gruppen beinhalten, mit Polyalkylenglykolderivaten erhalten werden können, deren Verwendung als Stabilisatoren organischen Materials gegen den schädigenden Einfluss von Licht, Sauerstoff und/oder Wärme, sowie die entsprechenden stabilisierten Zusammensetzungen.

Polyoxyalklyene mit endständigen 2,2,6,6-Tetramethyl-4-aminopiperidinyl-Resten sind aus dem US-Patent 5 210 195 bekannt und werden als Photostabilisatoren in photosensitiven Materialen vorgeschlagen.

Es besteht weiterhin Bedarf an neuen Stabilisatoren vom Typ der Bis-(2,2,6,6-Tetraalkyl-4-aminopiperidinyl)-polyoxyalkylenen mit verbesserten Gebrauchseigenschaften, insbesondere guter thermischer Beständigkeit als auch guter Löslichkeit in Wasser bzw wässrigen Lösungen oder allgemein in polaren Medien wie beispielsweise Polyamiden, Polyethern, Polyurethanen etc.

Es wurde nun gefunden, dass bestimmte Verbindungen, ausgehend von Polyalkylenglykolderivaten und 2,2,6,6-Tetramethylpiperidinen, sich überraschend gut als Stabilisatoren für insbesondere polares organisches Material eignen. Sie weisen auch eine hohe Temperaturbeständigkeit auf und erlauben somit in der Anwendung höhere Verarbeitungstemperaturen und damit einen höheren Durchsatz bei der weiteren Verarbeitung der so stabilisierten Produkte.

Gegenstand der Erfindung sind daher Verbindungen der Formel 1 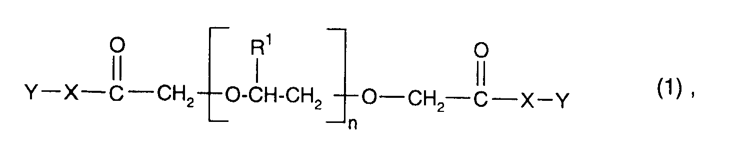 und Verbindungen der Formel 2 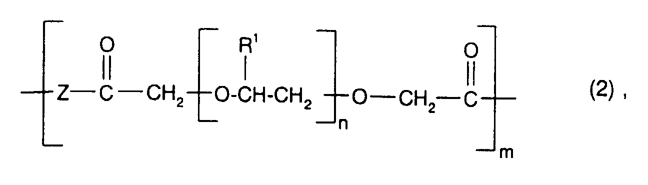 worin
R¹ H oder CH₃, und
Y die Gruppe 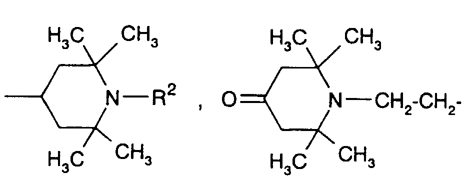 oder 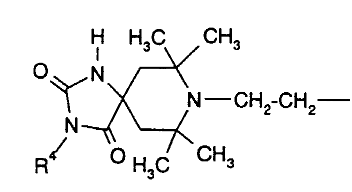 bedeutet,
X O oder NR³
R² H, C₁-C₂₀-Alkyl, C₁-C₂₀-Hydroxyalkyl, O-C₁-C₂₀-Alkyl, CO-C₁-C₂₀-Alkyl, O-C₅-C₈-Cydoalkyl, C₂-C₂₀-Alkenyl, C₂-C₂₀-Alkinyl, C₆-C₂₀-Aryl, CO-C₆-C₂₀-Aryl, C₇-C₂₀-Aralkyl, CO-C₇-C₂₀-Aralkyl, und
R³ H, C₁-C₂₀-Alkyl oder 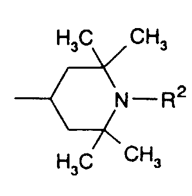 bedeuten, und
R⁴ H, C₁-C₂₀-Alkyl, CO-C₁-C₂₀-Alkyl, CO-C₆-C₂₀-Aryl oder CO-C₇-C₂₀-Aralkyl ist, und Z 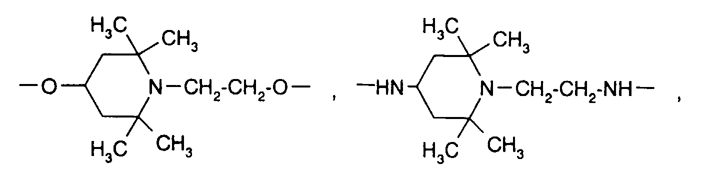 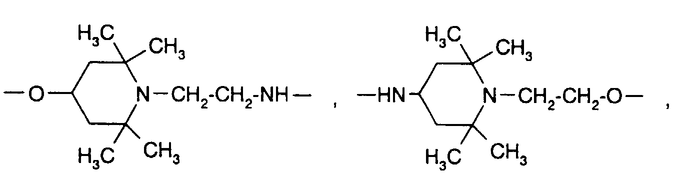 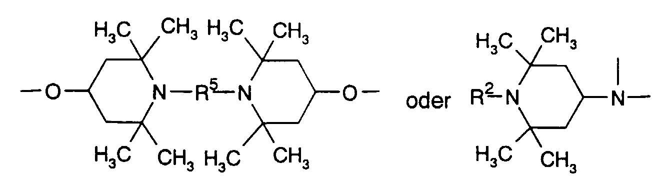 ist,
   und
R⁵ C₁-C₂₀-Alkylen, C₂-C₂₀-Alkenylen oder C₂-C₂₀-Alkinylen bedeuten, und
n eine Zahl von 1 bis 100 ist, und
m eine Zahl von 1 bis 100 ist.

R², R³ und R⁴ als C₁-C₂₀-Alkyl können geradkettig oder verzweigt sein und bedeuten z. B. Methyl, Ethyl, Propyl, Isopropyl, n-Butyl, sec-Butyl, Isobutyl, t-Butyl, 2-Ethylbutyl, n-Pentyl, Isopentyl, 1-Methylpentyl, 1,3-Dimethylbutyl, n-Hexyl, 1-Methylhexyl, n-Heptyl, Isoheptyl, 1,1,3,3-Tetramethylbutyl, 1-Methylheptyl, 3-Methylheptyl, n-Octyl, 2-Ethylhexyl, 1,1,3-Trimethylhexyl, 1,1,3,3-Tetramethylpentyl, Nonyl, Decyl, Undecyl, 1-Methylundecyl, Dodecyl, 1,1,3,3,5,5-Hexamethylhexyl u.s.w.
Die Alkylgruppen R², R³ und R⁴ weisen insbesondere 1 bis 12, R² und R⁴ vorzugsweise 1 bis 8 C-Atome auf.

R² als C₁-C₂₀-Hydroxyalkyl bedeutet, dass die geradkettige oder verzweigte Alkylkette 1 bis 20 C-Atome aufweist und dass sich die OH-Gruppe(n) an jeder beliebigen möglichen Position dieser Kette(n) befinden kann (können).

Vorzugsweise enthält der Rest 1 bis 3, insbesondere 1 oder 2 Hydroxy Gruppen, wie beispielsweise Hydroxymethyl, Hydroxyethyl, 3-Hydroxypropyl, 2-Hydroxypropyl, 4-Hydroxybutyl, 3-Hydroxybutyl, 2-Hydroxybutyl, 5-Hydroxypentyl, 4-Hydroxypentyl, 3-Hydroxypentyl, 2-Hydroxypentyl, 6-Hydroxyhexyl, 5-Hydroxyhexyl, 4-Hydroxyhexyl, 3-Hydroxyhexyl, 2-Hydroxyhexyl, 7-Hydroxyheptyl, 6-Hydroxyheptyl, 5-Hydroxyheptyl, 4-Hydroxyheptyl, 3-Hydroxyheptyl, 2-Hydroxyheptyl, 8-Hydroxyoctyl, 7-Hydroxyoctyl, 6-Hydroxyoctyl, 5-Hydroxyoctyl, 4-Hydroxyoctyl, 3-Hydroxyoctyl, 2-Hydroxyoctyl, 9-Hydroxynonyl, 10-Hydroxydecyl, 11-Hydroxyundecyl, 12-Hydroxydodecyl, 13-Hydroxytridecyl, 14-Hydroxytetradecyl, 15-Hydroxypentadecyl, 16-Hydroxyhexadecyl, 17-Hydroxyheptadecyl, 18-Hydroxyoctadecyl oder 20-Hydroxyeicosyl.

R² als C₅-C₈-Cycloalkyl bedeutet Cyclopentyl, Cyclohexyl, Cycloheptyl und Cyclooctyl. Bevorzugt ist Cyclohexyl.

R² als C₂-C₂₀-Alkenyl bedeutet z. B. Vinyl, 1-Propenyl, 2-Propenyl, 1-Butenyl, 2-Butenyl, 3-Butenyl u.s.w. Bevorzugt sind Alkenylgruppen mit 2 bis 12 C-Atomen, insbesondere mit 2 bis 8 C-Atomen.

R¹ als C₂-C₂₀-Alkinyl bedeutet z. B. Ethinyl, 1-Propinyl, 2-Propinyl, 1-Butinyl, 2-Butinyl, 3-Butinyl u.s.w. Bevorzugt haben solche Alkinylgruppen 2 bis 12 C-Atome, besonders bevorzugt sind solche mit 2 bis 8 C-Atomen.

R² und R⁴ als C₆-C₂₀-Aryl bedeuten z. B. Phenyl, Naphthyl, Anthracenyl, Phenanthrenyl. Bevorzugt ist Phenyl.

R² und R⁴ als Aralkyl bedeuten z. B. Benzyl, Phenethyl, 3-Phenylpropyl, α-Methylbenzyl oder α,α'-Dimethylbenzyl.
Bevorzugt ist Benzyl.

R⁵ als C₁-C₂₀-Alkylen bedeutet einen verzweigten oder unverzweigten Rest wie beispielsweise Methylen, Ethylen, Propylen, Trimethylen, Tetramethylen, Pentamethylen, Hexamethylen, Heptamethylen, Octamethylen, Decamethylen, Dodecamethylen oder Octadecamethylen. Bevorzugt ist C₁-C₁₂-Alkylen, insbesondere C₁-C₈-Alkylen.

R⁵ als C₂-C₂₀-Alkenylen bedeutet beispielsweise Vinylen, Methylvinylen, Octenylethylen oder Dodecenylethylen. Bevorzugt ist C₂-C₁₂-Alkenylen, insbesondere C₂-C₈-Alkenylen.

R⁵ als C₂-C₂₀-Alkinylen bedeutet beispielsweise Ethinylen, Propinylen, 1-Butinylen, 2-Butinylen, 1-Pentinylen, 2-Pentinylen, 1-Hexinylen, 2-Hexinylen, 3-Hexinylen u.s.w. Bevorzugt ist C₂-C₁₂-Alkinylen, insbesondere C₂-C₈-Alkinylen.

Bevorzugt sind Verbindungen der Formel 1 und 2, in denen R¹ Wasserstoff bedeutet.

Weiterhin bevorzugt sind Verbindungen der Formel 1 und 2, worin
R² H, C₁-C₁₂-Alkyl, C₁-C₁₂-Hydroxyalkyl, O-C₁-C₁₂-Alkyl, CO-C₁-C₁₂-Alkyl, O-C₅-C₈-Cydoalkyl, C₂-C₁₂-Alkenyl, C₂-C₁₂-Alkinyl, C₆-C₁₄-Aryl, CO-C₆-C₁₄-Aryl, C₇-C₁₅-Aralkyl oder CO-C₇-C₁₅-Aralkyl ist.

Besonders bevorzugt sind Verbindungen der Formel 1 und 2, worin
R² H, C₁-C₈-Alkyl, O-C₁-C₈-Alkyl, O-C₅-C₈-Cydoalkyl, C₂-C₈-Alkenyl oder C₂-C₈-Alkinyl ist.

Andere bevorzugte Verbindungen der Formel 1 sind solche, worin
R³ H, C₁-C₁₂-Alkyl oder 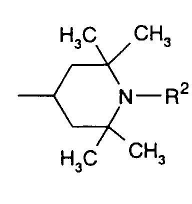 bedeuten, und
R⁴ H, C₁-C₁₂-Alkyl, CO-C₁-C₁₂-Alkyl, CO-C₆-C₁₄-Aryl oder CO-C₇-C₁₅-Aralkyl ist.

Ganz besonders bevorzugt sind Verbindungen der Formel 1, worin
R³ H oder 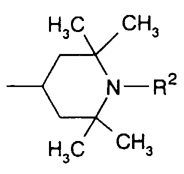 bedeutet, und
R⁴ H, C₁-C₈-Alkyl, CO-C₁-C₈-Alkyl, CO-Phenyl oder CO-Phenylalkyl ist.

Besonders bevorzugt sind Verbindungen der Formel 2, worin Z 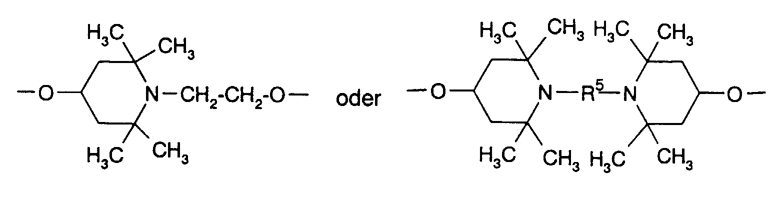 ist.

Bevorzugt sind Verbindungen der Formel 1 und 2, worin n eine Zahl von 1 bis 50 ist. Ebenso bevorzugt sind Verbindungen der Formel 2, worin m eine Zahl von 1 bis 50 ist. Bevorzugt sind die Verbindungen der Formel 1.

Besonders bevorzugt sind Verbindungen der Formel 1, worin
Y eine Gruppe 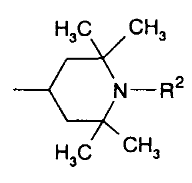 ist.

Ganz besonders bevorzugt sind Verbindungen der Formel 1, worin
Y eine Gruppe 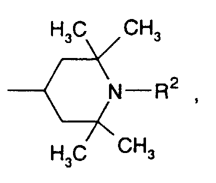
R² H oder C₁-C₄-Alkyl,
R³ H oder 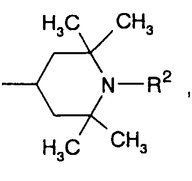 und
n eine Zahl von 5 bis 25 ist.

Die Darstellung der erfindungsgemässen Verbindungen erfolgt in an sich bekannter Weise, z. B. durch Umsetzung einer Polyalkylenglykoldisäure der Formel 3 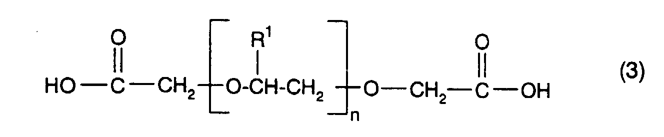 oder eines Derivates davon (z. B. Halogenid, Ester, Anhydrid) mit insbesondere 2 Moläquivalenten eines Amins oder Alkohols der Formel Y-NHR³ bzw. Y-OH (zur Herstellung von Verbindungen der Formel 1) bzw. mit vorzugsweise einem Moläquivalent einer difunktionellen Verbindung der Formel H-Z-H (zur Herstellung von Verbindungen der Formel 2). Die genannten Edukte sind entweder kommerziell erhältlich oder nach bekannten Verfahren leicht darstellbar. Im Falle eines sekundären Amins erfolgt die Darstellung des Amids zweckmässigerweise durch Reaktion des entsprechenden Polyalkylenglykoldisäurechlorids, das aus der Polyalkylenglykoldisäure und Thionylchlorid in bekannter Weise herstellbar ist, mit dem Amin. Überaus zweckmässig kann es auch sein, die entsprechenden erfindungsgemässen Verbindungen durch Umesterung aus den Polyalkylenglykolsäuredimethylestern zu erhalten. Derartige Umesterungen sind allgemein bekannt.

Die Umsetzungen erfolgen zweckmässig in einem inerten Lösungsmittel. Als Lösungsmittel können polare oder unpolare organische Lösungsmittel eingesetzt werden wie z. B. Kohlenwasserstoffe, halogenierte Kohlenwasserstoffe, Ester, Ether, Ketone, Amide, Nitrile, tert. Amine oder Sulfoxide; geeignet sind beispielsweise Toluol, Hexan, Cyclohexan, Ligroin, Petrolether und andere Kohlenwasserstoffgemische, Dimethylformamid, Tetrahydrofuran, Dioxan, Chloroform, Diethylether, Dimethylsulfoxid und Acetonitril; besonders bevorzugt sind Chloroform, Tetrahydrofuran und Toluol.

Die Temperatur kann während der Reaktion im Bereich von -20 bis 200°C liegen, zweckmässig beträgt die Temperatur 0 bis 180°C, vor allem 10 bis 140°C.

Die Temperatur der Reaktionsmischung kann für die Dauer der Reaktion im Siedebereich gehalten werden (Rückfluss). Dazu wird eine Lösemittel enthaltende Reaktionsmischung, im allgemeinen unter Normaldruck, zum Siedepunkt erwärmt und das verdampfte Lösemittel mit Hilfe eines geeigneten Kühlers kondensiert und wieder der Reaktionsmischung zugeführt.

Die Reaktionen können unter Ausschluss von Sauerstoff durchgeführt werden, beispielsweise durch Spülen mit einem Inertgas wie Argon; Sauerstoff stört jedoch nicht in jedem Fall, so dass die Reaktion auch ohne die genannte Massnahme durchgeführt werden kann.

Nach beendeter Reaktion kann die Aufarbeitung nach gängigen Methoden erfolgen; zweckmässig wird die Mischung zunächst mit Wasser verdünnt, beispielsweise indem das Reaktionsgemisch in das 1-4-fache Volumen (Eis-)Wasser gegeben wird; anschliessend kann das Produkt direkt abgetrennt oder extrahiert werden, zur Extraktion eignet sich z. B. Chloroform, Essigester oder Toluol. Wird extrahiert, so kann das Produkt in üblicher Weise durch Entfernen des Lösemittels isoliert werden; dies geschieht zweckmässig nach Trocknen der organischen Phase. Möglich ist auch das Einschalten weiterer Reinigungsschritte wie beispielsweise Waschen mit wässriger Natriumbicarbonatlösung, Dispergieren von Aktivkohle, chromatographieren mittels Kieselgels, Filtrieren, Umkristallisieren und/oder Destillieren.

Die erfindungsgemässen Verbindungen eignen sich besonders zum Stabilisieren von organischen Materialien gegen thermischen, oxidativen und aktinischen Abbau.

Beispiele für derartige Materialien sind:
1. Polymere von Mono- und Diolefinen, beispielsweise Polypropylen, Polyisobutylen, Polybuten-1, Poly-4-methylpenten-1, Polyisopren oder Polybutadien sowie Polymerisate von Cycloolefinen wie z. B. von Cyclopenten oder Norbornen; ferner Polyethylen (das gegebenenfalls vernetzt sein kann), z. B. Polyethylen hoher Dichte (HDPE), Polyethylen niederer Dichte (LDPE), lineares Polyethylen niederer Dichte (LLDPE), verzweigtes Polyethylen niederer Dichte (VLDPE).
   Polyolefine, d. h. Polymere von Monoolefinen, wie sie beispielhaft im vorstehenden Absatz erwähnt sind, insbesondere Polyethylen und Polypropylen, können nach verschiedenen Verfahren hergestellt werden, insbesondere nach den folgenden Methoden:
   a) radikalisch (gewöhnlich bei hohem Druck und hoher Temperatur).
   b) mittels Katalysator, wobei der Katalysator gewöhnlich ein oder mehrere Metalle der Gruppe IVb, Vb, Vlb oder VIII enthält. Diese Metalle besitzen gewöhnlich einen oder mehrere Liganden wie Oxide, Halogenide, Alkoholate, Ester, Ether, Amine, Alkyle, Alkenyle und/oder Aryle, die entweder π- oder σ-koordiniert sein können. Diese Metallkomplexe können frei oder auf Träger fixiert sein, wie beispielsweise auf aktiviertem Magnesiumchlorid, Titan(lll)chlorid, Aluminiumoxid oder Siliziumoxid. Diese Katalysatoren können im Polymerisationsmedium löslich oder unlöslich sein. Die Katalysatoren können als solche in der Polymerisation aktiv sein, oder es können weitere Aktivatoren verwendet werden, wie beispielsweise Metallalkyle, Metallhydride, Metallalkylhalogenide, Metallalkyloxide oder Metallalkyloxane, wobei die Metalle Elemente der Gruppen la, lla und/oder IIIa sind. Die Aktivatoren können beipielsweise mit weiteren Ester-, Ether-, Amin- oder Silylether-Gruppen modifiziert sein. Diese Katalysatorsysteme werden gewöhnlich als Phillips, Standard Oil Indiana, Ziegler(-Natta), TNZ (DuPont), Metallocen oder Single Site Katalysatoren (SSC) bezeichnet.
2. Mischungen der unter 1) genannten Polymeren, z. B. Mischungen von Polypropylen mit Polyisobutylen, Polypropylen mit Polyethylen (z. B. PP/HDPE, PP/LDPE) und Mischungen verschiedener Polyethylentypen (z. B. LDPE/HDPE).
3. Copolymere von Mono- und Diolefinen untereinander oder mit anderen Vinylmonomeren, wie z. B. Ethylen-Propylen-Copolymere, lineares Polyethylen niederer Dichte (LLDPE) und Mischungen desselben mit Polyethylen niederer Dichte (LDPE), Propylen--Buten-1-Copolymere, Propylen-lsobutylen-Copolymere, Ethylen-Buten-1-Copolymere, Ethylen-Hexen-Copolymere, Ethylen-Methylpenten-Copolymere, Ethylen-Hepten-Copolymere, Ethylen-Octen-Copolymere, Propylen-Butadien-Copolymere, Isobutylen-Isopren-Copolymere, Ethylen-Alkylacrylat-Copolymere, Ethylen-Alkylmethacrylat- Copolymere, Ethylen-Vinylacetat-Copolymere und deren Copolymere mit Kohlenstoffmonoxid, oder Ethylen-Acrylsäure-Copolymere und deren Salze (Ionomere), sowie Terpolymere von Ethylen mit Propylen und einem Dien, wie Hexadien, Dicyclopentadien oder Ethylidennorbornen; ferner Mischungen solcher Copolymere untereinander und mit unter 1) genannten Polymeren, z. B. Polypropylen/Ethylen-Propylen-Copolymere, LDPE/Ethylen-Vinylacetat-Copolymere, LDPE/Ethylen-Acrylsäure-Copolymere, LLDPE/Ethylen-Vinylacetat-Copolymere, LLDPE/Ethylen-Acrylsäure-Copolymere und alternierend oder statistisch aufgebaute Polyalkylen/Kohlenstoffmonoxid-Copolymere und deren Mischungen mit anderen Polymeren wie z. B. Polyamiden.
4. Kohlenwasserstoffharze (z. B. C₅-C₉) inklusive hydrierte Modifikationen davon (z. B. Klebrigmacherharze) und Mischungen von Polyalkylenen und Stärke.
5. Polystyrol, Poly-(p-methylstyrol), Poly-(a-methylstyrol).
6. Copolymere von Styrol oder α-Methylstyrol mit Dienen oder Acrylderivaten, wie z. B. Styrol-Butadien, Styrol-Acrylnitril, Styrol-Alkylmethacrylat, Styrol-Butadien-Alkylacrylat und -methacrylat, Styrol-Maleinsäureanhydrid, Styrol-Acrylnitril-Methylacrylat; Mischungen von hoher Schlagzähigkeit aus Styrol-Copolymeren und einem anderen Polymer, wie z. B. einem Polyacrylat, einem Dien-Polymeren oder einem Ethylen-Propylen-Dien-Terpolymeren; sowie Block-Copolymere des Styrols, wie z. B. Styrol-Butadien-Styrol, Styrol-Isopren-Styrol, Styrol-Ethylen/Butylen-Styrol oder Styrol-Ethylen/Propylen-Styrol.
7. Pfropfcopolymere von Styrol oder α-Methylstyrol, wie z. B. Styrol auf Polybutadien, Styrol auf Polybutadien-Styrol- oder Polybutadien-Acrylnitril-Copolymere, Styrol und Acrylnitril (bzw. Methacrylnitril) auf Polybutadien; Styrol, Acrylnitril und Methylmethacrylat auf Polybutadien; Styrol und Maleinsäureanhydrid auf Polybutadien; Styrol, Acrylnitril und Maleinsäureanhydrid oder Maleinsäureimid auf Polybutadien; Styrol und Maleinsäureimid auf Polybutadien, Styrol und Alkylacrylate bzw. Alkylmethacrylate auf Polybutadien, Styrol und Acrylnitril auf Ethylen-Propylen-Dien-Terpolymeren, Styrol und Acrylnitril auf Polyalkylacrylaten oder Polyalkylmethacrylaten, Styrol und Acrylnitril auf Acrylat-Butadien-Copolymeren, sowie deren Mischungen mit den unter 6) genannten Copolymeren, wie sie z. B. als sogenannte ABS-, MBS-, ASA- oder AES-Polymere bekannt sind.
8. Halogenhaltige Polymere, wie z. B. Polychloropren, Chlorkautschuk, chloriertes oder chlorsulfoniertes Polyethylen, Copolymere von Ethylen und chloriertem Ethylen, Epichlorhydrinhomo- und -copolymere, insbesondere Polymere aus halogenhaltigen Vinylverbindungen, wie z. B. Polyvinylchlorid, Polyvinylidenchlorid, Polyvinylfluorid, Polyvinylidenfluorid; sowie deren Copolymere, wie Vinylchlorid-Vinylidenchlorid, Vinylchlorid-Vinylacetat oder Vinylidenchlorid-Vinylacetat.
9. Polymere, die sich von α,β-ungesättigten Säuren und deren Derivaten ableiten, wie Polyacrylate und Polymethacrylate, mit Butylacrylat schlagzäh modifizierte Polymethylmethacrylate, Polyacrylamide und Polyacrylnitrile.
10. Copolymere der unter 9) genannten Monomeren untereinander oder mit anderen ungesättigten Monomeren, wie z. B. Acrylnitril-Butadien-Copolymere, Acrylnitril-Alkylacrylat-Copolymere, Acrylnitril-Alkoxyalkylacrylat-Copolymere, Acrylnitril-Vinylhalogenid-Copolymere oder Acrylnitril-Alkylmethacrylat-Butadien-Terpolymere.
11. Polymere, die sich von ungesättigten Alkoholen und Aminen bzw. deren Acylderivaten oder Acetalen ableiten, wie Polyvinylalkohol, Polyvinylacetat, -stearat, -benzoat, -maleat, Polyvinylbutyral, Polyallylphthalat, Polyallylmelamin; sowie deren Copolymere mit in Punkt 1 genannten Olefinen.
12. Homo- und Copolymere von cyclischen Ethern, wie Polyalkylenglykole, Polyethylenoxyd, Polypropylenoxyd oder deren Copolymere mit Bisglycidylethern.
13. Polyacetale, wie Polyoxymethylen, sowie solche Polyoxymethylene, die Comonomere, wie z. B. Ethylenoxid, enthalten; Polyacetale, die mit thermoplastischen Polyurethanen, Acrylaten oder MBS modifiziert sind.
14. Polyphenylenoxide und -sulfide und deren Mischungen mit Styrolpolymeren oder Polyamiden.
15. Polyurethane, die sich von Polyethern, Polyestern und Polybutadienen mit endständigen Hydroxylgruppen einerseits und aliphatischen oder aromatischen Polyisocyanaten andererseits ableiten, sowie deren Vorprodukte.
16. Polyamide und Copolyamide, die sich von Diaminen und Dicarbonsäuren und/oder von Aminocarbonsäuren oder den entsprechenden Lactamen ableiten, wie Polyamid 4, Polyamid 6, Polyamid 6/6, 6/10, 6/9, 6/12, 4/6, 12/12, Polyamid 11, Polyamid 12, aromatische Polyamide ausgehend von m-Xylol, Diamin und Adipinsäure; Polyamide, hergestellt aus Hexamethylendiamin und Iso- und/oder Terephthalsäure und gegebenenfalls einem Elastomer als Modifikator, z. B. Poly-2,4,4-trimethylhexamethylenterephthalamid oder Poly-m-phenylenisophthalamid. Block-Copolymere der vorstehend genanntenPolyamide mit Polyolefinen, Olefin-Copolymeren, Ionomeren oder chemisch gebundenen oder gepfropften Elastomeren; oder mit Polyethern, wie z. B. mit Polyethylenglykol, Polypropylenglykol oder Polytetramethylenglykol. Ferner mit EPDM oder ABS modifizierte Polyamide oder Copolyamide; sowie während der Verarbeitung kondensierte Polyamide ("RIM-Polyamidsysteme").
17. Polyharnstoffe, Polyimide, Polyamid-imide und Polybenzimidazole.
18. Polyester, die sich von Dicarbonsäuren und Dialkoholen und/oder von Hydroxycarbonsäuren oder den entsprechenden Lactonen ableiten, wie Polyethylenterephthalat, Polybutylenterephthalat, Poly-1,4-dimethylolcyclohexanterephthalat, Polyhydroxybenzoate, sowie Block-Polyether-ester, die sich von Polyethern mit Hydroxylendgruppen ableiten; ferner mit Polycarbonaten oder MBS modifizierte Polyester.
19. Polycarbonate und Polyestercarbonate.
20. Polysulfone, Polyethersulfone und Polyetherketone.
21. Vernetzte Polymere, die sich von Aldehyden einerseits und Phenolen, Harnstoff oder Melamin andererseits ableiten, wie Phenol-Formaldehyd-, Harnstoff-Formaldehyd- und Melamin-Formaldehydharze.
22. Trocknende und nicht-trocknende Alkydharze.
23. Ungesättigte Polyesterharze, die sich von Copolyestern gsättigter und ungesättigter Dicarbonsäuren mit mehrwertigen Alkoholen, sowie Vinylverbindungen als Vernetzungsmittel ableiten, wie auch deren halogenhaltige, schwerbrennbare Modifikationen.
24. Vernetzbare Acrylharze, die sich von substituierten Acrylsäureestern ableiten, wie z. B. von Epoxyacrylaten, Urethan-acrylaten oder Polyester-acrylaten.
25. Alkydharze, Polyesterharze und Acrylatharze, die mit Melaminharzen, Harnstoffharzen, Isocyanaten, Isocyanuraten, Polyisocyanaten oder Epoxidharzen vernetzt sind.
26. Vernetzte Epoxidharze, die sich von Polyepoxiden ableiten, z. B. von Bis-glycidylethern oder von cycloaliphatischen Diepoxiden.
27. Natürliche Polymere, wie Cellulose, Naturkautschuk, Gelatine, sowie deren polymerhomolog chemisch abgewandelte Derivate, wie Celluloseacetate, -propionate und -butyrate, bzw. die Celluloseether, wie Methylcellulose; sowie Kolophoniumharze und Derivate.
28. Mischungen (Polyblends) der vorgenannten Polymeren, wie z. B. PP/EPDM, Polyamid/EPDM oder ABS, PVC/EVA, PVC/ABS, PVC/MBS, PC/ABS, PBTP/ABS, PC/ASA, PC/PBT, PVC/CPE, PVC/Acrylate, POM/thermoplastisches PUR, PC/thermoplastisches PUR, POM/Acrylat, POM/MBS, PPO/HIPS, PPO/PA 6.6 und Copolymere, PA/HDPE, PA/PP, PA/PPO.

Weitere Gegenstände der Erfindung sind daher Zusammensetzungen enthaltend a) ein gegen oxidativen, thermischen oder/und aktinischen Abbau/Aufbau empfindliches organisches Material und b) mindestens eine erfindungsgemässe Verbindung gemäss Anspruch 1, sowie die Verwendung der erfindungsgemässen Verbindungen zum Stabilisieren von organischem Material gegen oxidativen, thermischen oder aktinischen Abbau/Aufbau.
Die Erfindung umfasst ebenfalls ein Verfahren zum Stabilisieren von organischem Material gegen thermischen, oxidativen oder/und aktinischen Abbau/Aufbau, dadurch gekennzeichnet, dass man diesem Material mindestens eine erfindungsgemässe Verbindung zusetzt.

Von besonderem Interesse ist die Verwendung der erfindungsgemässen Verbindungen als Stabilisatoren in synthetischen organischen Polymeren sowie entsprechende Zusammensetzungen.

Vorzugsweise handelt es sich bei den zu schützenden organischen Materialien um natürliche, halbsynthetische oder bevorzugt synthetische organische Materialien. Besonders bevorzugt sind synthetische organische Polymere oder Gemische solcher Polymere, insbesondere thermoplastische Polymere wie Polyester und Polyamide. Ebenfalls besonders bevorzugte organische Materialien sind Überzugszusammensetzungen. Im Sinne der Erfindung vorteilhaft zu stabilisierende Überzugszusammensetzungen sind beispielsweise beschrieben in Ullmann's Encyclopedia of Industrial Chemistry, 5. Ed., Vol. A 18, Seiten 359 - 464, VCH Verlagsgesellschaft, Weinheim 1991.

Von besonderem Interesse ist die Verwendung der erfindungsgemäßen Verbindungen als Stabilisatoren für Überzüge, beispielsweise für Lacke. Gegenstand der Erfindung sind daher auch solche Zusammensetzungen, deren Komponente A ein filmbildendes Bindemittel ist.

Das erfindungsgemäße Überzugsmittel enthält vorzugsweise auf 100 Gew.-Teile festes Bindemittel A 0,01-10 Gew.-Teile, insbesondere 0,05-10 Gew.-Teile, vor allem 0,1-5 Gew.-Teile des erfindungsgemäßen Stabilisators B.

Auch hier sind Mehrschichtsysteme möglich, wobei die Konzentration der Komponente B in der Deckschicht höher sein kann, beispielsweise 1 bis 15 Gew.-Teile, vor allem 3 bis 10 Gew.-Teile B auf 100 Gew.-Teile festes Bindemittel A.

Die Verwendung der erfindungsgemässen Verbindungen als Stabilisator in Überzügen bringt den zusätzlichen Vorteil mit sich, daß der Delaminierung, d. h. dem Abblättern des Überzuges vom Substrat, vorgebeugt wird. Dieser Vorteil kommt insbesondere bei metallischen Substraten zum tragen, auch bei Mehrschichtsystemen auf metallischen Substraten.

Als Bindemittel (Komponente A) kommen prinzipiell alle in der Technik gebräuchlichen in Betracht, beispielsweise solche, wie sie beschrieben sind in Ullmann's Encyclopedia of Industrial Chemistry, 5. Ed., Vol. A 18, Seiten 368 - 426, VCH Verlagsgesellschaft, Weinheim 1991. Allgemein handelt es sich um ein filmbildendes Bindemittel basierend auf einem thermoplastischen oder thermohärtbaren Harz, vorwiegend auf einem thermohärtbaren Harz. Beispiele hierfür sind Alkyd-, Acryl-, Polyester-, Phenol-, Melamin-, Epoxid-, Polyurethanharze und deren Gemische.

Komponente A kann ein kalt aushärtbares oder ein heiß aushärtbares Bindemittel sein, wobei die Zugabe eines Härtungskatalysators vorteilhaft sein kann. Geeignete Katalysatoren, die die Aushärtung des Bindemittels beschleunigen, sind beispielsweise beschrieben in Ullmann's Encyclopedia of Industrial Chemistry, Vol. A 18, S. 469, VCH Verlagsgesellschaft, Weinheim 1991.

Bevorzugt sind Überzugsmittel, worin die Komponente A ein Bindemittel aus einem funktionellen Acrylatharz und einem Vernetzer ist.

Beispiele von Überzugsmitteln mit speziellen Bindemitteln sind:
1. Lacke auf Basis von kalt- oder heiß-vernetzbaren Alkyd-, Acrylat-, Polyester-, Epoxid- oder Melaminharzen oder Mischungen solcher Harze, gegebenenfalls mit Zusatz eines Härtungskatalysators;
2. Zweikomponenten-Polyurethanlacke auf Basis von hydroxylgruppenhaltigen Acrylat-, Polyester- oder Polyetherharzen und aliphatischen oder aromatischen Isocyanaten, Isocyanuraten oder Polyisocyanaten;
3. Einkomponenten-Polyurethanlacke auf Basis von blockierten Isocyanaten, Isocyanuraten oder Polyisocyanaten, die während des Einbrennens deblockiert werden;
4. Einkomponenten-Polyurethanlacke auf Basis von aliphatischen oder aromatischen Urethanen oder Polyurethanen und hydroxylgruppenhaltigen Acrylat-, Polyester- oder Polyetherharzen;
5. Einkomponenten-Polyurethanlacke auf Basis von aliphatischen oder aromatischen Urethanen oder Polyurethanen mit freien Amingruppen in der Urethanstruktur und Melaminharzen oder Polyetherharzen, gegebenenfalls mit Zusatz eines Härtungskatalysators;
6. Zweikomponentenlacke auf Basis von (Poly)ketiminen und aliphatischen oder aromatischen Isocyanaten, Isocyanuraten oder Polyisocyanaten;
7. Zweikomponentenlacke auf Basis von (Poly)ketiminen und einem ungesättigten Acrylatharz oder einem Polyacetoacetatharz oder einem Methacrylamidoglykolatmethylester;
8. Zweikomponentenlacke auf Basis von carboxyl- oder aminogruppenhaltigen Polyacrylaten und Polyepoxiden;
9. Zweikomponentenlacke auf Basis von anhydridgruppenhaltigen Acrylatharzen und einer Polyhydroxy- oder Polyaminokomponente;
10. Zweikomponentenlacke auf Basis von acrylathaltigen Anhydriden und Polyepoxiden;
11. Zweikomponentenlacke auf Basis von (Poly)oxazolinen und anhydridgruppenhaltigen Acrylatharzen oder ungesättigten Acrylatharzen oder aliphatischen oder aromatischen Isocyanaten, Isocyanuraten oder Polyisocyanaten;
12. Zweikomponentenlacke auf Basis von ungesättigten Polyacrylaten und Polymalonaten;
13. thermoplastische Polyacrylatlacke auf Basis von thermoplastischen Acrylatharzen oder fremdvernetzenden Acrylatharzen in Kombination mit veretherten Melaminharzen;
14. Lacksysteme auf Basis von siloxanmodifizierten oder fluormodifizierten Acrylatharzen.

Das erfindungsgemäße Überzugsmittel enthält vorzugsweise neben den Komponenten A und B als Komponente C mindestens ein weiteres Lichtschutzmittel, insbesondere einen UV-Absorber, z. B. einen aus den Gruppen 2.1 - 2.4, 2.7 oder 2.8 der weiter unten folgenden Liste von Costabilisatoren. Von besonderem technischem Interesse ist dabei der Zusatz von 2-Hydroxyphenyl-2H-benztriazolen und/oder 2-Hydroxyphenyl-1,3,5-triazinen.

Das zusätzliche Lichtschutzmittel (Komponente C) wird vorzugsweise in einer Menge von 0,05-5 Gew.-Teilen auf 100 Gew.-Teile des festen Bindemittels verwendet.

Das Überzugsmittel kann außer den Komponenten A, B und gegebenenfalls C weitere Komponenten enthalten, z. B. Lösungsmittel, Pigmente, Farbstoffe, Weichmacher, Stabilisatoren, Thixotropiemittel, Trocknungskatalysatoren und/oder Verlaufhilfsmittel. Mögliche Komponenten sind beispielsweise solche, wie sie in Ullmann's Encyclopedia of Industrial Chemistry, 5. Ed., Vol. A 18, Seiten 429 - 471, VCH Verlagsgesellschaft, Weinheim 1991 beschrieben sind.

Mögliche Trocknungskatalysatoren beziehungsweise Härtungskatalysatoren sind beispielsweise organische Metallverbindungen, Amine, aminogruppenhaltige Harze oder/und Phosphine. Organische Metallverbindungen sind z. B. Metallcarboxylate, insbesondere solche der Metalle Pb, Mn, Co, Zn, Zr oder Cu, oder Metallchelate, insbesondere solche der Metalle Al, Ti oder Zr oder Organometallverbindungen wie z. B. Organozinnverbindungen.

Beispiele für Metallcarboxylate sind die Stearate von Pb, Mn oder Zn, die Octoate von Co, Zn oder Cu, die Naphthenate von Mn und Co oder die entsprechenden Linoleate, Resinate oder Tallate.

Beispiele für Metallchelate sind die Aluminium-, Titan- oder Zirkonium-Chelate von Acetylaceton, Ethylacetylacetat, Salicylaldehyd, Salicylaldoxim, o-Hydroxyacetophenon oder Ethyl-trifluoracetylacetat und die Alkoxide dieser Metalle.

Beispiele für Organozinnverbindungen sind Dibutylzinnoxid, Dibutylzinn-dilaurat oder Dibutylzinn-dioctoat.

Beispiele für Amine sind vor allem tertiäre Amine, wie z. B. Tributylamin, Triethanolamin, N-Methyl-diethanolamin, N-Dimethylethanolamin, N-Ethylmorpholin, N-Methylmorpholin oder Diazabicyclooctan (Triethylendiamin) sowie deren Salze. Weitere Beispiele sind quaternäre Ammoniumsalze, wie z. B. Trimethylbenzylammoniumchlorid.

Aminogruppenhaltige Harze sind gleichzeitig Bindemittel und Härtungskatalysator. Beispiel hierfür sind aminogruppenhaltige Acrylat-copolymere.

Als Härtungskatalysator können auch Phosphine verwendet werden, wie z. B. Triphenylphosphin.

Bei den erfindungsgemäßen Überzugsmitteln kann es sich auch um strahlenhärtbare Überzugsmittel handeln. In diesem Fall besteht das Bindemittel im wesentlichen aus monomeren oder oligomeren Verbindungen mit ethylenisch ungesättigten Bindungen (Präpolymeren), die nach der Applikation durch aktinische Strahlung gehärtet, d. h. in eine vernetzte, hochmolekulare Form überführt werden. Handelt es sich um ein UV-härtendes System, enthält dies in der Regel zusätzlich einen Photoinitiator. Entsprechende Systeme sind in der oben genannten Publikation, Ullmann's Encyclopedia of Industrial Chemistry, 5. Ed., Vol. A 18, Seiten 451 - 453, beschrieben. In strahlenhärtbaren Überzugsmitteln können die erfindungsgemäßen Stabilisatoren auch ohne Zusatz von sterisch gehinderten Aminen eingesetzt werden.

Die erfindungsgemäßen Überzugsmittel können auf beliebige Substrate aufgebracht werden, beispielsweise auf Metall, Holz, Kunststoff oder keramische Materialien. Vorzugsweise werden sie beim Lackieren von Automobilen als Decklack verwendet. Besteht der Decklack aus zwei Schichten, wovon die untere Schicht pigmentiert ist und die obere Schicht nicht pigmentiert ist, so kann das erfindungsgemäße Überzugsmittel für die obere oder die untere Schicht oder für beide Schichten verwendet werden, vorzugsweise jedoch für die obere Schicht.

Die erfindungsgemäßen Überzugsmittel können auf die Substrate nach den üblichen Verfahren aufgebracht werden, beispielsweise durch Streichen, Besprühen, Gießen, Tauchen oder Elektrophorese; s. a. Ullmann's Encyclopedia of Industrial Chemistry, 5. Ed., Vol. A 18, Seiten 491 - 500.

Die Härtung der Überzüge kann - je nach dem Bindemittelsystem - bei Raumtemperatur oder durch Erwärmen erfolgen. Vorzugsweise härtet man die Überzüge bei 50-150°C, Pulverlacke auch bei höheren Temperaturen.

Die erfindungsgemäß erhaltenen Überzüge weisen eine hervorragende Beständigkeit gegen schädigende Einflüsse von Licht, Sauerstoff und Wärme auf; insbesondere ist auf die gute Licht- und Witterungsbeständigkeit der so erhaltenen Überzüge, beispielsweise Lacke, hinzuweisen.

Gegenstand der Erfindung ist daher auch ein Überzug, besonders ein Lack, der durch Zusatz mindestens einer erfindungsgemässen Verbindung gegen schädigende Einflüsse von Licht, Sauerstoff und Wärme stabilisiert ist. Der Lack ist vorzugsweise ein Decklack für Automobile. Die Erfindung beinhaltet weiterhin ein Verfahren zum Stabilisieren eines Überzuges auf Basis organischer Polymere gegen Schädigung durch Licht, Sauerstoff und/oder Wärme, dadurch gekennzeichnet, daß man dem Überzugsmittel mindestens eine erfindungsgemässe Verbindung beimischt, sowie die Verwendung der erfindungsgemässen Verbindungen in Überzugsmitteln als Stabilisatoren gegen Schädigung durch Licht, Sauerstoff und/oder Wärme.

Die Überzugsmittel können ein organisches Lösungsmittel oder Lösungsmittelgemisch enthalten, in dem das Bindemittel löslich ist. Das Überzugsmittel kann aber auch eine wässrige Lösung oder Dispersion sein. Das Vehikel kann auch ein Gemisch eines organischen Lösungmittels und Wasser sein. Das Überzugsmittel kann auch ein feststoffreicher Lack (high solids Lack) sein oder kann lösungsmittelfrei sein (z. B. Pulverlack). Pulverlacke sind beispielsweise solche, wie sie in Ullmann's Encyclopedia of Industrial Chemistry, 5. Ed., A 18, Seiten 438 - 444 beschrieben sind. Der Pulverlack kann auch in Form eines Pulver-Slurry, d. h. einer Dispersion des Pulvers bevorzugt in Wasser vorliegen.

Die Pigmente können anorganische, organische oder metallische Pigmente sein. Vorzugsweise enthalten die erfindungsgemäßen Überzugsmittel keine Pigmente und werden als Klarlack verwendet.

Ebenfalls bevorzugt ist der Einsatz des Überzugsmittels als Decklack für Anwendungen in der Automobilindustrie, besonders als pigmentierte oder unpigmentierte Deckschicht des Lackes. Die Verwendung für darunter liegende Schichten ist jedoch auch möglich.

Weitere mit den erfindungsgemässen Verbindungen zu stabilisierende Materialien sind photographische Materialien. Unter solchen sind insbesondere solche zu verstehen, die in Research Disclosure 1990, 31429 (Seiten 474 - 480) für die photographische Reproduktion und andere Reproduktionstechniken beschrieben sind.

Allgemein werden die erfindungsgemässen Verbindungen dem zu stabilisierenden Material in Mengen von 0,01 bis 10 %, bevorzugt 0,01 bis 5 %, insbesondere 0,01 bis 2 %, bezogen auf das Gesamtgewicht der stabilisierten Zusammensetzung, zugesetzt. Besonders bevorzugt ist der Einsatz der erfindungsgemäßen Verbindungen in Mengen von 0,05 bis 1,5 %, vor allem 0,1 bis 0,5 %.

Die Einarbeitung in die Materialien kann beispielsweise durch Einmischen oder Aufbringen der erfindungsgemässen Verbindungen und gegebenenfalls weiterer Additive nach den in der Technik üblichen Methoden erfolgen. Handelt es sich um Polymere, insbesondere synthetische Polymere, kann die Einarbeitung vor oder während der Formgebung, oder durch Aufbringen der gelösten oder dispergierten Verbindung auf das Polymere, gegebenenfalls unter nachträglichem Verdunsten des Lösungsmittels erfolgen. Im Fall von Elastomeren können diese auch als Latices stabilisiert werden. Eine weitere Möglichkeit der Einarbeitung der erfindungsgemässen Verbindungen in Polymere besteht in deren Zugabe vor, während oder unmittelbar nach der Polymerisation der entsprechenden Monomeren bzw. vor der Vernetzung. Die erfindungsgemässen Verbindungen können dabei als solche, aber auch in encapsulierter Form (z. B in Wachsen, Ölen oder Polymeren) zugesetzt werden. Im Falle der Zugabe vor oder während der Polymerisation können die erfindungsgemässen Verbindungen auch als Regulator für die Kettenlänge der Polymere (Kettenabbrecher) wirken.

Die erfindungsgemässen Verbindungen können auch in Form eines Masterbatches, der diese Verbindung beispielsweise in einer Konzentration von 2,5 bis 25 Gew.-% enthält, den zu stabilisierenden Kunststoffen zugesetzt werden.

Zweckmäßig kann die Einarbeitung der erfindungsgemässen Verbindungen nach folgenden Methoden erfolgen:
- als Emulsion oder Dispersion (z. B. zu Latices oder Emulsionspolymeren),
- als Trockenmischung während des Vermischens von Zusatzkomponenten oder Polymermischungen,
- durch direktes Zugeben in die Verarbeitungsapparatur (z. B. Extruder, Innenmischer usw.),
- als Lösung oder Schmelze.

Erfindungsgemäße Polymerzusammensetzungen können in verschiedener Form angewendet bzw. zu verschiedenen Produkten verarbeitet werden, z. B. als (zu) Folien, Fasern, Bändchen, Formmassen, Profilen, oder als Bindemittel für Lacke, Klebstoffe oder Kitte.

Neben den Erfindungsgemässen Verbindungen können die erfindungsgemäßen Zusammensetzungen als zusätzliche Komponente (c) ein oder mehrere herkömmliche Additive enthalten, wie beispielsweise die folgenden:
1. Antioxidantien
   1.1. Alkylierte Monophenole, z. B. 2,6-Di-tert-butyl-4-methylphenol, 2-Butyl-4,6-dimethylphenol, 2,6-Di-tert-butyl-4-ethylphenol, 2,6-Di-tert-butyl-4-n-butylphenol, 2,6-Di-tert-butyl-4-iso-butylphenol, 2,6-Di-cyclopentyl-4-methylphenol, 2-(a-Methylcyclohexyl)-4,6-dimethylphenol, 2,6-Di-octadecyl-4-methylphenol, 2,4,6-Tri-cyclohexylphenol, 2,6-Di-tert-butyl-4-methoxymethylphenol, lineare oder in der Seitenkette verzweigte Nonylphenole wie z. B. 2,6-Di-nonyl-4-methylphenol, 2,4-Dimethyl-6-(1'-methyl-undec-1'-yl)-phenol, 2,4-Dimethyl-6-(1'-methyl-heptadec-1'-yl)-phenol, 2,4-Dimethyl-6-(1'-methyl-tridec-1'-yl)-phenol und Mischungen davon.
   1.2. Alkylthiomethylphenole, z. B. 2,4-Di-octylthiomethyl-6-tert-butylphenol, 2,4-Di-octylthiomethyl-6-methylphenol, 2,4-Di-octylthiomethyl-6-ethylphenol, 2,6-Di-dodecylthiomethyl-4-nonylphenol.
   1.3. Hydrochinone und alkylierte Hydrochinone, z. B. 2,6-Di-tert-butyl-4-methoxyphenol, 2,5-Di-tert-butyl-hydrochinon, 2,5-Di-tert-amyl-hydrochinon, 2,6-Diphenyl-4-octadecyloxyphenol, 2,6-Di-tert-butyl-hydrochinon, 2,5-Di-tert-butyl-4-hydroxyanisol, 3,5-Di-tert-butyl-4-hydroxyanisol, 3,5-Di-tert-butyl-4-hydroxyphenyl-stearat, Bis(3,5-di-tert-butyl-4-hydroxyphenyl)adipat.
   1.4. Tocopherole, z. B. α-Tocopherol, β-Tocopherol, γ-Tocopherol, δ-Tocopherol und Mischungen davon (Vitamin E).
   1.5. Hydroxylierte Thiodiphenylether, z. B. 2,2'-Thio-bis(6-tert-butyl-4-methylphenol), 2,2'-Thio-bis(4-octylphenol), 4,4'-Thio-bis(6-tert-butyl-3-methylphenol), 4,4'-Thio-bis-(6-tert-butyl--2-methylphenol), 4,4'-Thio-bis(3,6-di-sec.-amylphenol), 4,4'-Bis(2,6-dimethyl-4-hydroxyphenyl)-disulfid.
   1.6. Alkyliden-Bisphenole, z. B. 2,2'-Methylen-bis(6-tert-butyl-4-methylphenol), 2,2'-Methylen-bis(6-tert-butyl-4-ethylphenol), 2,2'-Methylen-bis[4-methyl-6-(α-methylcyclohexyl)-phenol], 2,2'-Methylen-bis(4-methyl-6-cyclohexylphenol), 2,2'-Methylen-bis(6-nonyl-4-methylphenol), 2,2'-Methylen-bis(4,6-di-tert-butylphenol), 2,2'-Ethyliden-bis(4,6-di-tert-butylphenol), 2,2'-Ethyliden-bis(6-tert-butyl-4-isobutylphenol), 2,2'-Methylen-bis[6-(α-methylbenzyl)-4-nonylphenol], 2,2'-Methylen-bis[6-(α,α-dimethylbenzyl)-4-nonylphenol], 4,4'-Methylen-bis(2,6-di-tert-butylphenol), 4,4'-Methylen-bis(6-tert-butyl-2-methylphenol), 1,1-Bis(5-tert-butyl-4-hydroxy-2-methylphenyl)-butan, 2,6-Bis(3-tert-butyl-5-methyl-2-hydroxybenzyl)-4-methylphenol, 1,1,3-Tris(5-tert-butyl-4-hydroxy-2-methylphenyl)-butan, 1,1-Bis(5-tert-butyl-4-hydroxy-2-methyl-phenyl)-3-n-dodecylmercaptobutan, Ethylenglycol-bis[3,3-bis(3'-tert-butyl-4'-hydroxyphenyl)-butyrat], Bis(3-tert-butyl-4-hydroxy-5-methyl-phenyl)-dicyclopentadien, Bis[2-(3'-tert-butyl-2'-hydroxy-5'-methyl-benzyl)-6-tert-butyl-4-methyl-phenyl]-terephthalat, 1,1-Bis(3,5-dimethyl-2-hydroxyphenyl)-butan, 2,2-Bis(3,5-di-tert-butyl-4-hydroxyphenyl)-propan, 2,2-Bis (5-tert-butyl-4-hydroxy-2-methylphenyl) -4-n-dodecylmercapto-butan, 1,1,5,5--Tetra-(5-tert-butyl-4-hydroxy-2-methylphenyl)-pentan.
   1.7. O-, N- und S-Benzylverbindungen, z. B. 3,5,3',5'-Tetra-tert-butyl-4,4'-dihydroxydibenzylether, Octadecyl-4-hydroxy-3,5-dimethylbenzyl-mercaptoacetat, Tridecyl-4-hydroxy-3,5-di-tert-butylbenzyl-mercaptoacetat, Tris(3,5-di-tert-butyl-4-hydroxybenzyl)-amin, Bis(4-tert-butyl-3-hydroxy-2,6-dimethylbenzyl)-dithioterephthalat, Bis(3,5-di-tert-butyl-4-hydroxybenzyl)sulfid, Isooctyl-3,5-di-tert-butyl-4-hydroxybenzyl-mercaptoacetat.
   1.8. Hydroxybenzylierte Malonate, z. B. Dioctadecyl-2,2-bis(3,5-di-tert-butyl-2-hydroxybenzyl)-malonat, Di-octadecyl-2-(3-tert-butyl-4-hydroxy-5-methylbenzyl)-malonat, Di-dodecylmercaptoethyl-2,2-bis (3,5-di-tert-butyl-4-hydroxybenzyl) -malonat, Di-[4-(1,1,3,3-tetramethylbutyl)-phenyl]-2,2-bis(3 ,5-di-tert-butyl-4-hydroxybenzyl) -malonat.
   1.9. Hydroxybenzyl-Aromaten, z. B. 1 ,3,5-Tris(3,5-di-tert-butyl-4-hydroxybenzyl)-2,4,6-trimethylbenzol, 1 ,4-Bis(3,5-di-tert-butyl-4-hydroxybenzyl)-2,3,5,6-tetramethylbenzol, 2,4,6-Tris(3,5-di-tert-butyl-4-hydroxybenzyl)-phenol.
   1.10. Triazinverbindungen, z. B. 2,4-Bis-octylmercapto-6-(3,5-di-tert-butyl-4-hydroxyanilino)-1,3,5-triazin, 2-Octylmercapto-4,6-bis(3,5-di-tert-butyl-4-hydroxyanilino)-1,3,5-triazin, 2-Octylmercapto-4,6-bis(3,5-di-tert-butyl-4-hydroxyphenoxy)-1 ,3,5-triazin, 2,4,6-Tris(3,5-di-tert-butyl-4-hydroxyphenoxy)-1,2,3-triazin, 1 ,3,5-Tris(3,5-di-tert-butyl-4-hydroxybenzyl)-isocyanurat, 1,3,5-Tris (4-tert-butyl-3-hydroxy-2,6-dimethylbenzyl)-isocyanurat, 2,4,6-Tris(3,5-di-tert-butyl-4-hydroxyphenylethyl)-,3,5-triazin, 1 ,3,5-Tris(3,5-di-tert-butyl-4-hydroxyphenylpropionyl)-hexahydro-1,3,5-triazin, 1,3,5-Tris(3,5-dicyclohexyl-4-hydroxybenzyl)-isocyanurat.
   1.11. Benzylphosphonate, z. B. Dimethyl-2,5-di-tert-butyl-4-hydroxybenzylphosphonat, Di-ethyl-3,5-di-tert-butyl-4-hydroxybenzylphosphonat, Dioctadecyl-3,5-di-tert-butyl-4-hydroxybenzylphosphonat, Dioctadecyl-5-tert-butyl-4-hydroxy-3-methylbenzylphosphonat, Ca-Salz des 3,5-Di-tert-butyl-4-hydroxybenzyl-phosphonsäure-monoethylesters.
   1.12. Acylaminophenole, z. B. 4-Hydroxy-laurinsäureanilid, 4-Hydroxystearinsäureanilid, N-(3,5-di-tert-butyl-4-hydroxyphenyl)-carbaminsäureoctylester.
   1.13. Ester der β-(3,5-Di-tert-butyl-4-hydroxyphenyl)-propionsäure mit ein- oder mehrwertigen Alkoholen, wie z. B. mit Methanol, Ethanol, n-Octanol, i-Octanol, Octadecanol, 1,6-Hexandiol, 1,9-Nonandiol, Ethylenglycol, 1,2-Propandiol, Neopentylglycol, Thiodiethylenglycol, Diethylenglycol, Triethylenglycol, Pentaerythrit, Tris(hydroxyethyl)-isocyanurat, N,N'-Bis(hydroxyethyl)-oxalsäurediamid, 3-Thiaundecanol, 3-Thiapentadecanol, Trimethylhexandiol, Trimethylolpropan, 4-Hydroxymethyl-1-phospha-2,6,7-trioxabicyclo-[2.2.2]-octan.
   1.14. Ester der β-(5-tert-Butyl-4-hydroxy-3-methylphenyl)-propionsäure mit ein- oder mehrwertigen Alkoholen, wie z. B. mit Methanol, Ethanol, n-Octanol, i-Octanol, Octadecanol, 1,6-Hexandiol, 1,9-Nonandiol, Ethylenglycol, 1,2-Propandiol, Neopentylglycol, Thiodiethylenglycol, Diethylenglycol, Triethylenglycol, Pentaerythrit, Tris(hydroxyethyl)-isocyanurat, N,N'-Bis(hydroxyethyl)-oxalsäurediamid, 3-Thiaundecanol, 3-Thiapentadecanol, Trimethylhexandiol, Trimethylolpropan, 4-Hydroxymethyl-1-phospha-2,6,7-trioxabicyclo-[2.2.2]-octan.
   1.15. Ester der β-(3,5-Dicyclohexyl-4-hydroxyphenyl)-propionsäure mit ein- oder mehrwertigen Alkoholen, wie z. B. mit Methanol, Ethanol, Octanol, Octadecanol, 1,6-Hexandiol, 1,9-Nonandiol, Ethylenglycol, 1,2-Propandiol, Neopentylglycol, Thiodiethylenglycol, Diethylenglycol, Triethylenglycol, Pentaerythrit, Tris(hydroxyethyl)-isocyanurat, N,N'-Bis(hydroxyethyl)oxalsäurediamid, 3-Thiaundecanol, 3-Thiapentadecanol, Trimethylhexandiol, Trimethylolpropan, 4-Hydroxymethyl-1-phospha-2,6,7-trioxabicyclo-[2.2.2]-octan.
   1.16. Ester der 3,5-Di-tert-butyl-4-hydroxyphenylessigsäure mit ein- oder mehrwertigen Alkoholen, wie z. B. mit Methanol, Ethanol, Octanol, Octadecanol, 1,6-Hexandiol, 1,9-Nonandiol, Ethylenglycol, 1,2-Propandiol, Neopentylglycol, Thiodiethylenglycol, Diethylenglycol, Triethylenglycol, Pentaerythrit, Tris(hydroxyethyl)-isocyanurat, N,N'-Bis(hydroxyethyl)--oxalsäurediamid, 3-Thiaundecanol, 3-Thiapentadecanol, Trimethylhexandiol, Trimethylolpropan, 4-Hydroxymethyl-1-phospha-2,6,7-trioxabicyclo-[2.2.2]-octan.
   1.17. Amide der β-(3,5-Di-tert-butyl-4-hydroxyphenyl)-propionsäure, wie z. B. N,N'-Bis(3,5-di-tert-butyl-4-hydroxyphenylpropionyl)-hexamethylendiamin, N,N'-Bis(3,5-di-tert-butyl-4-hydroxyphenylpropionyl)-trimethylendiamin, N,N'-Bis (3,5-di-tert-butyl-4-hydroxyphenylpropionyl)-hydrazin.
   1.18. Ascorbinsäure (Vitamin C).
   1.19. Aminische Antioxidantien, wie z. B. N,N'-Di-isopropyl-p-phenylendiamin, N,N'-Di-sec-butyl-p-phenylendiamin, N,N'-Bis(1 ,4-dimethyl-pentyl)-p-phenylendiamin, N,N'-Bis(1-ethyl-3-methyl-pentyl)-p-phenylendiamin, N,N'-Bis(1-methyl-heptyl)-p-phenylendiamin, N,N'-Dicyclohexyl-p-phenylendiamin, N,N'-Diphenyl-p-phenylendiamin, N,N'-Di-(naphthyl-2)-p-phenylendiamin, N-lsopropyl-N'-phenyl-p-phenylendiamin, N-(1,3-Dimethyl-butyl)-N'-phenyl-p-phenylendiamin, N-(1-Methyl-heptyl)-N'-phenyl-p-phenylendiamin, N-Cyclohexyl-N'-phenyl-p-phenylendiamin, 4-(p-Toluol-sulfonamido)-diphenylamin, N, N'-Dimethyl-N,N'-di-sec-butyl-p-phenylendiamin, Diphenylamin, N-Allyldiphenylamin, 4-lsopropoxy-diphenylamin, N-Phenyl-1-naphthylamin, N-(4-tert-Octylphenyl)-1-naphthylamin, N-Phenyl-2-naphthylamin, octyliertes Diphenylamin, z. B. p,p'-Di-tert-octyldiphenylamin, 4-n-Butylaminophenol, 4-Butyrylamino--phenol, 4-Nonanoylamino-phenol, 4-Dodecanoylamino-phenol, 4-Octadecanoylamino--phenol, Di-(4-methoxyphenyl)-amin, 2,6-Di-tert-butyl-4-dimethylamino-methyl-phenol, 2,4'-Di-amino-diphenylmethan, 4,4'-Diamino-diphenylmethan, N,N,N',N'-Tetramethyl-4,4'-diamino-diphenylmethan, 1,2-Di-[(2-methyl-phenyl)-amino]-ethan, 1,2-Di-(phenylamino)-propan, (o--Tolyl)-biguanid, Di-[4-(1',3'-dimethyl-butyl)-phenyl]amin, tert-octyliertes N-Phenyl-1-naphthylamin, Gemisch aus mono- und dialkylierten tert-Butyl/tert-Octyldiphenylaminen, Gemisch aus mono- und dialkylierten Nonyldiphenylaminen, Gemisch aus mono- und dialkylierten Dodecyldiphenylaminen, Gemisch aus mono- und dialkylierten Isopropyl/Isohexyl--diphenylaminen, Gemische aus mono- und dialkylierten tert-Butyldiphenylaminen, 2,3-Di-hydro-3,3-dimethyl-4H-1,4-benzothiazin, Phenothiazin, Gemisch aus mono- und dialkylierten tert-Butyl/tert-Octyl-phenothiazinen, Gemisch aus mono- und dialkylierten tert-Octylphenothiazinen, N-Allylphenothiazin, N,N,N',N'-Tetraphenyl-1,4-diaminobut-2-en, N,N-Bis--(2,2,6,6-tetramethyl-piperidin-4-yl-hexamethylendiamin, Bis-(2,2,6,6-tetramethylpiperidin-4-yl)-sebacat, 2,2,6,6-Tetramethylpiperidin-4-on, 2,2,6,6-Tetramethylpiperidin-4-ol.
2. UV-Absorber und Lichtschutzmittel
   2.1. 2-(2'-Hydroxyphenyl)-benzotriazole, wie z. B. 2-(2'-Hydroxy-5'-methylphenyl)-benzotriazol, 2-(3',5'-Di-tert-butyl-2'-hydroxyphenyl)-benzotriazol, 2-(5'-tert-Butyl-2'-hydroxyphenyl)-benzotriazol, 2-(2'-Hydroxy-5'-(1,1,3,3-tetramethylbutyl)phenyl)-benzotriazol, 2-(3',5'-Di-tert-butyl-2'-hydroxyphenyl)-5-chlor-benzotriazol, 2-(3'-tert-Butyl- 2'-hydroxy-5'-methylphenyl)-5-chlor-benzotriazol, 2-(3'-sec-Butyl-5'-tert-butyl-2'-hydroxyphenyl)-benzotriazol, 2-(2'-Hydroxy-4'-octoxyphenyl)-benzotriazol, 2-(3',5'-Di-tert-amyl-2'-hydroxyphenyl)-benzotriazol, 2-(3',5'--Bis(α,α-dimethylbenzyl)-2'-hydroxyphenyl)-benzotriazol, Mischung aus 2-(3'-tert-Butyl-2'-hydroxy-5'-(2-octyloxycarbonylethyl)phenyl)-5-chlor-benzotriazol, 2-(3'-tert-Butyl-5'-[2-(2-ethylhexyloxy)-carbonylethyl]-2'-hydroxyphenyl)-5-chlor-benzotriazol, 2-(3'-tert-Butyl-2'-hydroxy-5'-(2-methoxycarbonylethyl)phenyl)-5-chlor-benzotriazol, 2-(3'-tert-Butyl-2'-hydroxy--5'-(2-methoxycarbonylethyl) phenyl)-benzotriazol, 2-(3'-tert-Butyl-2'-hydroxy-5'-(2-octyloxycarbonylethyl)phenyl)-benzotriazol, 2-(3'-tert-Butyl-5'-[2-(2-ethylhexyloxy)carbonylethyl]-2'-hydroxyphenyl)-benzotriazol, 2-(3'-Dodecyl-2'-hydroxy-5'-methylphenyl)-benzotriazol, und 2-(3'-tert-Butyl-2'-hydroxy-5'-(2-isooctyloxycarbonylethyl)phenyl-benzotriazol, 2,2'-Methylen-bis[4-(1,1,3,3-tetramethylbutyl)-6-benzotriazol-2-yl-phenol] ; Umesterungsprodukt von 2-[3'-tert-Butyl-5'-(2-methoxycarbonylethyl)-2'-hydroxy-phenyl]-benzotriazol mit Polyethylenglycol 300;mit R = 3'-tert-Butyl-4'-hydroxy-5'-2H-benzotriazol-2-yl-phenyl.
   2.2. 2-Hydroxybenzophenone, wie z. B. das 4-Hydroxy-, 4-Methoxy-, 4-Octoxy-, 4-Decyloxy-, 4-Dodecyloxy-, 4-Benzyloxy-, 4,2',4'-Trihydroxy-, 2'-Hydroxy-4,4'-dimethoxy-Derivat.
   2.3. Ester von gegebenenfalls substituierten Benzoesäuren, wie z. B. 4-tert-Butyl-phenylsalicylat, Phenylsalicylat, Octylphenyl-salicylat, Dibenzoylresorcin, Bis(4-tert-butylbenzoyl)-resorcin, Benzoylresorcin, 3,5-Di-tert-butyl-4-hydroxybenzoesäure-2,4-di-tert-butylphenylester, 3,5-Di-tert-butyl-4-hydroxybenzoesäurehexadecylester, 3,5-Di-tert-butyl-4-hydroxybenzoesäure-octadecylester, 3,5-Di-tert-butyl-4-hydroxybenzoesäure-2-methyl-4,6-di-tert-butylphenylester.
   2.4. Acrylate, wie z. B. α-Cyan-β,β-diphenylacrylsäure-ethylester bzw. -isooctylester, α-Carbomethoxy-zimtsäuremethylester, a-Cyano-β-methyl-p-methoxy-zimtsäuremethylester bzw. -butylester, a-Carbomethoxy-p-methoxy-zimtsäure-methylester, N-(β-Carbomethoxy-β--cyanovinyl)-2-methyl-indolin.
   2.5. Nickelverbindungen, wie z. B. Nickelkomplexe des 2,2'-Thio-bis[4-(1,1,3,3-tetramethylbutyl)-phenols], wie der 1:1- oder der 1:2-Komplex, gegebenenfalls mit zusätzlichen Liganden, wie n-Butylamin, Triethanolamin oder N-Cyclohexyl-diethanolamin, Nickeldibutyl dithiocarbamat, Nickelsalze von 4-Hydroxy-3,5-di-tert-butylbenzylphosphonsäure-monoalkylestern, wie vom Methyl- oder Ethylester, Nickelkomplexe von Ketoximen, wie von 2-Hydroxy-4-methyl-phenyl-undecylketoxim, Nickelkomplexe des 1-Phenyl-4-lauroyl-5-hydroxypyrazols, gegebenenfalls mit zusätzlichen Liganden.
   2.6. Weitere sterisch gehinderte Amine, wie z. B. Bis(2,2,6,6-tetramethyl-piperidin-4-yl)-sebacat, Bis(2,2,6,6-tetramethyl-piperidin-4-yl)-succinat, Bis(1,2,2,6,6-pentamethylpiperidin--4-yl)-sebacat, Bis(1-octyloxy-2,2,6,6-tetramethylpiperidin-4-yl)-sebacat, n-Butyl-3,5-di-tert--butyl-4-hydroxybenzyl-malonsäure-bis(1,2,2,6,6-pentamethylpiperidyl)-ester, Kondensationsprodukt aus 1-Hydroxyethyl-2,2,6,6-tetramethyl-4-hydroxypiperidin und Bernsteinsäure, Kondensationsprodukt aus N,N'-Bis(2,2,6,6-Tetramethyl-4-piperidyl)-hexamethylendiamin und 4-tert-Octylamino-2,6-dichlor-1,3,5-s-triazin, Tris(2,2,6,6-tetramethyl-4-piperidyl)--nitrilotriacetat, Tetrakis(2,2,6,6-tetramethyl-4-piperidyl)-1,2,3,4-butantetraoat, 1,1'-(1,2-Ethandiyl)-bis(3,3,5,5-tetramethyl-piperazinon), 4-Benzoyl-2,2,6,6-tetramethylpiperidin, 4-Stearyloxy-2,2,6,6-tetramethylpiperidin, Bis(1 ,2,2,6,6-pentamethylpiperidyl)-2-n-butyl-2-(2-hydroxy-3,5-di-tert-butylbenzyl)-malonat, 3-n-Octyl-7,7,9,9-tetramethyl-1,3,8-triazaspiro[4.5]decan-2,4-dion, Bis(1-octyloxy-2,2,6,6-tetramethylpiperidyl)-sebacat, Bis(1-octyloxy-2,2,6,6-tetramethylpiperidyl)-succinat, Kondensationsprodukt aus N,N'-Bis(2,2,6,6--tetramethyl-4-piperidyl)-hexamethylendiamin und 4-Morpholino-2,6-dichlor-1,3,5-triazin, Kondensationsprodukt aus 2-Chlor-4,6-di-(4-n-butylamino-2,2,6,6-tetramethylpiperidyl)-1,3,5-triazin und 1,2-Bis(3-aminopropylamino)ethan, Kondensationsprodukt aus 2-Chlor-4,6-di-(4-n-butylamino-1,2,2,6,6-pentamethylpiperidyl)-,3,5-triazin und 1 ,2-Bis(3-aminopropylamino)-äthan, 8-Acetyl-3-dodecyl-7,7,9,9-tetramethyl-1,3,8-triazaspiro[4.5]decan-2,4-dion, 3-Dodecyl-1-(2,2,6,6-tetramethyl-4-piperidyl)pyrrolidin-2,5-dion, 3-Dodecyl-1-(1,2,2,6,6-pentamethyl-4-piperidyl)-pyrrolidin-2,5-dion, Gemisch von 4-Hexadecyloxy- und 4-Stearyloxy-2,2,6,6-tetramethylpiperidin, Kondenstionsprodukt aus N,N'-Bis(2,2,6,6-tetramethyl-4-piperidyl)-hexamethylendiamin und 4-Cyclohexylamino-2,6-dichlor-1,3,5-triazin, Kondensationsprodukt aus 1,2-Bis(3-aminopropylamino)-ethan und 2,4,6-trichlor-1,3,5-triazin sowie 4-Butylamino-2,2,6,6-tetramethyl-piperidin (CAS Reg. No. [136504-96-6]); N-(2,2,6,6-tetramethyl-4-piperidyl)-n-dodecylsuccinimid, N-(1,2,2,6,6-pentamethyl-4-piperidyl)-n-dodecylsuccinimid, 2-Undecyl-7,7,9,9-tetramethyl-1-oxa-3,8-diaza-4-oxo-spiro[4,5]decan, Umsetzungsprodukt von 7,7,9,9-Tetramethyl-2-cycloundecyl-1-oxa-3,8-diaza-4-oxospiro[4,5]decan und Epichlorhydrin.
   2.7. Oxalsäurediamide, wie z. B. 4,4'-Di-octyloxy-oxanilid, 2,2'-Diethoxy-oxanilid, 2,2'-Di-octyloxy-5,5'-di-tert-butyl-oxanilid, 2,2'-Di-dodecyloxy-5,5'-di-tert-butyl-oxanilid, 2-Ethoxy-2'-ethyl-oxanilid, N,N'-Bis(3-dimethylaminopropyl)-oxalamid, 2-Ethoxy-5-tert-butyl-2'-ethyloxanilid und dessen Gemisch mit 2-Ethoxy-2'-ethyl-5,4'-di-tert-butyl-oxanilid, Gemische von o- und p-Methoxy- sowie von o- und p-Ethoxy-di-substituierten Oxaniliden.
   2.8. 2-(2-Hydroxyphenyl)-1,3,5-triazine, wie z. B. 2,4,6-Tris(2-hydroxy-4-octyloxyphenyl)-1,3,5-triazin, 2-(2-Hydroxy-4-octyloxyphenyl)-4,6-bis(2,4-dimethylphenyl)-1,3,5-triazin, 2-(2,4-Dihydroxyphenyl)-4,6-bis(2,4-dimethylphenyl)-1,3,5-triazin, 2,4-Bis(2-hydroxy-4-propyloxyphenyl)-6-(2,4-dimethylphenyl)-1,3,5-triazin, 2-(2-Hydroxy-4-octyloxyphenyl)-4,6-bis(4-methylphenyl)-1,3,5-triazin, 2-(2-Hydroxy-4-dodecyloxyphenyl)-4,6-bis(2,4-dimethylphenyl)-1,3,5-triazin, 2-(2-Hydroxy-4-tridecyloxyphenyl)-4,6-bis(2,4-dimethylphenyl)-1,3,5-triazin, 2-[2-Hydroxy-4-(2-hydroxy-3-butyloxy-propyloxy)phenyl]-4,6-bis(2,4-dimethylphenyl)-1,3,5-triazin, 2-[2-Hydroxy-4-(2-hydroxy-3-octyloxy-propyloxy)phenyl]-4,6-bis(2,4-dimethylphenyl)-1,3,5-triazin, 2-[4-(dodecyloxy/tridecyloxy-2-hydroxypropoxy)-2-hydroxy-phenyl]-4,6-bis(2,4-dimethylphenyl)-1,3,5-triazin, 2-[2-Hydroxy-4- (2-hydroxy-3-dodecyloxy-propoxy) phenyl]-4,6-bis(2,4-dimethylphenyl)-1,3,5-triazin, 2-(2-Hydroxy-4-hexyloxy)phenyl-4,6-diphenyl-1,3,5-triazin, 2-(2-Hydroxy-4-methoxyphenyl)-4,6-diphenyl-1,3,5-triazin, 2,4,6-Tris[2-hydroxy-4-(3-butoxy-2-hydroxy-propoxy) phenyl]-1,3,5-triazin, 2- (2-Hydroxyphenyl)-4-(4-methoxyphenyl)-6-phenyl-1 ,3,5-triazin.
3. Metalldesaktivatoren, wie z. B. N,N'-Diphenyloxalsäurediamid, N-Salicylal-N'-salicyloylhydrazin, N,N'-Bis(salicyloyl)-hydrazin, N,N'-Bis(3,5-di-tert-butyl-4-hydroxyphenylpropionyl)-hydrazin, 3-Salicyloylamino-1,2,4-triazol, Bis(benzyliden)-oxalsäuredihydrazid, Oxanilid, Isophthalsäure-dihydrazid, Sebacinsäure-bis-phenylhydrazid, N,N'-Diacetyl-adipinsäuredihydrazid, N,N'-Bis-salicyloyl-oxalsäure-dihydrazid, N,N'-Bis-salicyloyl-thiopropionsäuredihydrazid.
4. Phosphite und Phosphonite, wie z. B. Triphenylphosphit, Diphenylalkylphosphite, Phenyldialkylphosphite, Tris(nonylphenyl)-phosphit, Trilaurylphosphit, Trioctadecylphosphit, Distearylpentaerythritdiphosphit, Tris(2,4-di-tert-butylphenyl)-phosphit, Diisodecylpentaerythrit-diphosphit, Bis(2,4-di-tert-butylphenyl)-pentaerythritdiphosphit, Bis-(2,6-di-tert-butyl-4-methylphenyl)-pentaerythritdiphosphit, Bis-isodecyloxy-pentaerythritdiphosphit, Bis(2,4-di-tert-butyl-6-methylphenyl)-pentaerythritdiphosphit, Bis-(2,4,6-tri-tert-butylphenyl)-pentaerythritdiphosphit, Tristearyl-sorbit-triphosphit, Tetrakis(2,4-di-tert-butylphenyl)-4,4'-biphenylen-diphosphonit, 6-Isooctyloxy-2,4,8,10-tetra-tert-butyl-12H-dibenz[d,g]-1,3,2-dioxaphosphocin, 6-Fluor-2,4,8,10-tetra-tert-butyl-12-methyl-dibenz[d,g]-1,3,2-dioxaphosphocin, Bis-(2,4-di-tert-butyl-6-methylphenyl)-methylphosphit, Bis(2,4-di-tert-butyl-6-methylphenyl)-ethylphosphit.
5. Hydroxylamine wie z. B. N,N-Dibenzylhydroxylamin, N,N-diethylhydroxylamin, N,N-Dioctylhydroxylamin, N,N-Dilaurylhydroxylamin, N,N-Ditetradecylhydroxylamin, N,N-Dihexadecylhydroxylamin, N,N-Dioctadecylhydroxylamin, N-Hexadecyl-N-octadecylhydroxylamin, N-Heptadecyl-N-octadecylhydroxylamin, N,N-Dialkylhydroxylamin aus hydrierten Talgfettaminen.
6. Nitrone wie z. B. N-Benzyl-alpha-phenyl-nitron, N-Ethyl-alpha-methyl-nitron, N-Octyl--alpha-heptyl-nitron, N-Lauryl-alpha-undecyl-nitron, N-Tetradecyl-alpha-tridecyl-nitron, N-Hexadecyl-alpha-pentadecyl-nitron, N-Octadecyl-alpha-heptadecyl-nitron, N-Hexadecylalpha-heptadecyl-nitron, N-Ocatadecyl-alpha-pentadecyl-nitron, N-Heptadecyl-alphaheptadecyl-nitron, N-Octadecyl-alpha-hexadecyl-nitron, Nitrone abgeleitet von N,N-Dialkylhydroxylaminen hergestellt aus hydrierten Talgfettaminen.
7. Thiosynergisten wie z. B. Thiodipropionsäure-di-laurylester oder Thiodipropionsäure-distearylester.
8. Peroxidzerstörende Verbindungen, wie z. B. Ester der β-Thio-dipropionsäure, beispielsweise der Lauryl-, Stearyl-, Myristyl- oder Tridecylester, Mercaptobenzimidazol, das Zinksalz des 2-Mercaptobenzimidazols, Zink-dibutyl-dithiocarbamat, Dioctadecyldisulfid, Pentaerythrittetrakis(β-dodecylmercapto)-propionat.
9. Polyamidstabilisatoren, wie z. B. Kupfersalze in Kombination mit Jodiden und/oder Phosphorverbindungen und Salze des zweiwertigen Mangans.
10. Basische Co-Stabilisatoren, wie z. B. Melamin, Polyvinylpyrrolidon, Dicyandiamid, Triallylcyanurat, Harnstoff-Derivate, Hydrazin-Derivate, Amine, Polyamide, Polyurethane, Alkaliund Erdalkalisalze höherer Fettsäuren, beispielsweise Ca-Stearat, Zn-Stearat, Mg-Behenat, Mg-Stearat, Na-Ricinoleat, K-Palmitat, Antimonbrenzcatechinat oder Zinnbrenzcatechinat.
11. Nukleierungsmittel, wie z. B. anorganische Stoffe wie z. B. Talk, Metalloxide wie Titandioxid oder Magnesiumoxid, Phosphate, Carbonate oder Sulfate von vorzugsweise Erdalkalimetallen; organische Verbindungen wie Mono- oder Polycarbonsäuren sowie ihre Salze wie z. B. 4-tert-Butylbenzoesäure, Adipinsäure, Diphenylessigsäure, Natriumsuccinat oder Natriumbenzoat; polymere Verbindungen wie z. B. ionische Copolymerisate ("Ionomere").
12. Füllstoffe und Verstärkungsmittel, wie z. B. Calciumcarbonat, Silikate, Glasfasern, Glaskugeln, Asbest, Talk, Kaolin, Glimmer, Bariumsulfat, Metalloxide und -hydroxide, Ruß, Graphit, Holzmehl und Mehle oder Fasern anderer Naturprodukte, synthetische Fasern.
13. Sonstige Zusätze, wie z. B. Weichmacher, Gleitmittel, Emulgatoren, Pigmente, Rheologieadditive, Katalysatoren, Verlaufshilfsmittel, Optische Aufheller, Flammschutzmittel, Antistatika, Treibmittel.
14. Benzofuranone bzw. Indolinone, wie z. B. in US-A-4325863, US-A-4338244, US-A-5175312, US-A-5216052, US-A-5252643, DE-A-4316611, DE-A-4316622, DE-A-4316876, EP-A-0589839 oder EP-A-0591102 beschrieben, oder 3-[4-(2-Acetoxyethoxy)phenyl]-5,7-di-tert-butyl-benzofuran-2-on, 5,7-Di-tert-butyl-3-[4-(2-stearoyloxyethoxy)phenyl]-benzofuran-2-on, 3,3'-Bis[5,7-di-tert-butyl-3-(4-[2-hydroxyethoxy]phenyl)-benzofuran-2-on], 5,7-Di-tert-butyl-3-(4-ethoxyphenyl)benzofuran-2-on, 3-(4-Acetoxy-3,5-dimethylphenyl)-5,7-di-tert-butyl--benzofuran-2-on, 3-(3,5-Dimethyl-4-pivaloyloxy-phenyl)-5,7-di-tert-butyl-benzofuran-2-on.

Diese zusätzlichen Additive werden zweckmäßig in Mengen von 0,1-10, beispielsweise 0,2-5 Gew.-%, bezogen auf das zu stabilisierende Polymer, eingesetzt.

Die nachfolgenden Beispiele illustrieren die Erfindung weiter. Alle Angaben in Teilen oder Prozenten, in den Beispielen ebenso wie in der übrigen Beschreibung sowie in den Patentansprüchen, beziehen sich auf das Gewicht, sofern nichts anderes angegeben ist. In den Beispielen werden die folgenden Abkürzungen verwendet:
- ax:: axial
- CDCl₃:: Deuterochloroform
- d. Th.:: der Theorie
- eq:: equatorial
- ¹H-NMR:: Magnetische Kernresonanz des Nuklids ¹H
- MALDI - MS:: Matrix Assisted Laser Desorption/lonization - Massenspektroskopie
- Mₙ:: Zahlenmittel der Molmasse (Einheit g/Mol)
- M_{w}:: Massenmittel der Molmasse (Einheit g/Mol)
- PEG:: Polyethylengylkol(600)
- THF:: Tetrahydrofuran

### Herstellungsbeispiele

### Beispiel 1: PEG-Disäure-bis-(3,3,5,5-tetramethylpiperidinylamid)

60 g (0,1 Mol) PEG-Disäure und 32 g (0,2 Mol) 4-Amino-2,2,6,6-tetramethylpiperidin werden unter Inertgas auf 180°C erwärmt. Man lässt bei dieser Temperatur ca. 5 Stunden reagieren, wobei das sich bei der Reaktion bildende Wasser via Destillationsaufsatz überdestilliert. Man kühlt anschliessend auf Raumtemperatur ab und löst das Reaktionsprodukt in Chloroform vollständig auf. Nun wäscht man die organische Phase mit kochsalzgesättigter Natronlauge. Die organische Phase wird anschliessend mit Natriumsulfat getrocknet. Das Lösungsmittel wird am Rotationsverdampfer entfernt. Letzte Reste des Lösungsmittels werden anschliessend im Hochvakuum bei 90°C entfernt.
Man erhält 60 g (75 % d. Th.) einer viskosen Flüssigkeit, die in Wasser sehr gut löslich ist.

| Mikroanalyse: | | |
|---|---|---|
| | berechnet | gefunden |
| C | 63,2 % | 59,7 % |
| H | 10,2 % | 10,0% |
| N | 7,0 % | 7,0 % |

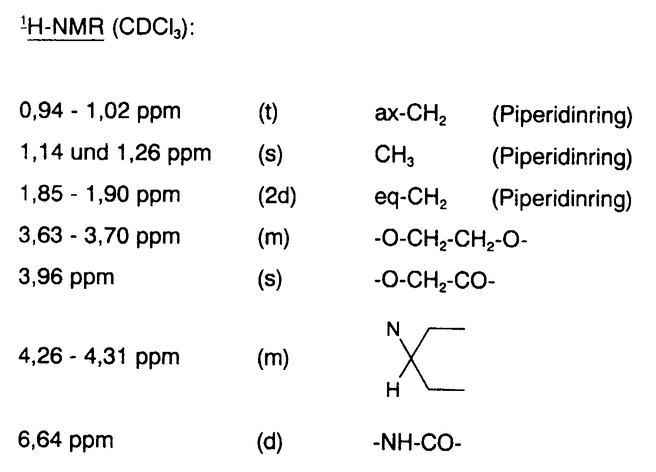

MALDI-MS: Mₙ = 796 M_{w} = 867

### Beispiel 2: PEG-Disäure-bis-(3,3,4,5,5-pentamethylpiperidinylamid)

110 g (183 mMol) PEG-Disäure und 70 g (412 mMol) 4-Amino-1,2,2,6,6-pentamethylpiperidin werden wie in Beispiel 1 beschrieben zur Reaktion gebracht und aufgearbeitet. Man erhält 105 g (58 % d. Th.) einer viskosen Flüssigkeit, die in Wasser gut löslich ist.

| Mikroanalyse: | | |
|---|---|---|
| | berechnet | gefunden |
| C | 60,1 % | 59,6 % |
| H | 9,8 % | 9,8 % |
| N | 6,4 % | 7,1 % |

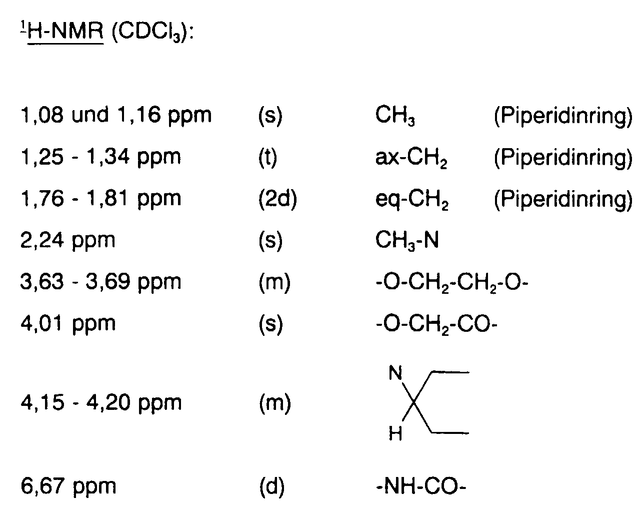

MALDI-MS: Mₙ = 847 M_{w} = 911

### Beispiel 3: PEG-Disäure-bis-(3,3,4,5,5-pentamethylpiperidin-1-ylester)

### 3a) PEG-Disäuredimethylester

In einem 1 Liter Rundkolben, mit Magnetrührer und Rückflusskühler versehen, werden unter Argon 300 g (0,5 Mol) PEG-Disäure in 300 ml Methanol gelöst. Man gibt anschliessend 10 ml konzentrierte Schwefelsäure hinzu und kocht die Lösung über Nacht am Rückfluss. Nach dem Abkühlen auf Raumtemperatur giesst man die Lösung auf Eis. Anschliessend wird mit Chloroform ausgezogen und die organische Phase mit kochsalzgesättigter Natronlauge gewaschen. Die organische Phase wird getrocknet und das Lösungsmittel vollständig entfernt.
Man erhält 250 g (83 % d. Th.) einer klaren, viskosen Flüssigkeit.

| Mikroanalyse: | | |
|---|---|---|
| | berechnet | gefunden |
| C | 51,8 % | 52,8 % |
| H | 8,3 % | 8,6 % |

### ¹H-NMR (CDCl₃):

- 3,64 - 3,76 ppm: (m, 40 H) -O-CH₂-CH₂-O-
- 3,77 ppm: (s, 6 H) COOCH₃
- 4,23 ppm: (s, 4 H) -O-CH₂-CO-

### 3b) PEG-Disäure-bis-(3,3,4,5,5-pentamethylpiperidin-1-ylester)

In einem 1 Liter Rundkolben, mit Magnetrührer und Destillationsaufsatz versehen, werden unter Argon 120 g (0,2 Mol) des unter 3a) hergestellten Dimethylesters, 69 g (0,4 Mol) 4-Hydroxy-1,2,2,6,6-pentamethylpiperidin, 7 g Dibutylzinnoxid und 600 ml Xylol vorgelegt. Man erwärmt auf 140°C und belässt die Lösung solange bei dieser Temperatur, bis kein Methanol mehr überdestilliert. Anschliessend wird stärker erwärmt, so dass auch langsam Xylol überdestilliert. Nachdem ca. 100 ml Xylol abdestilliert sind, wird auf Raumtemperatur abgekühlt und der verbleibende Rest an Lösungsmittel anschliessend am Rotationsverdampfer entfernt. Man nimmt den Rückstand in Chloroform auf und wäscht die organische Phase mit kochsalzgesättigter Natronlauge. Die organische Phase wird getrocknet, abfiltriert und das Lösungsmittel am Rotationsverdampfer entfernt. Man erhält 100 g (60 % d. Th.) einer viskosen Flüssigkeit.

| Mikroanalyse: | | |
|---|---|---|
| | berechnet | gefunden |
| C | 59,1 % | 58,4 % |
| H | 9,5 % | 9,9 % |
| N | 2,6 % | 2,5 % |

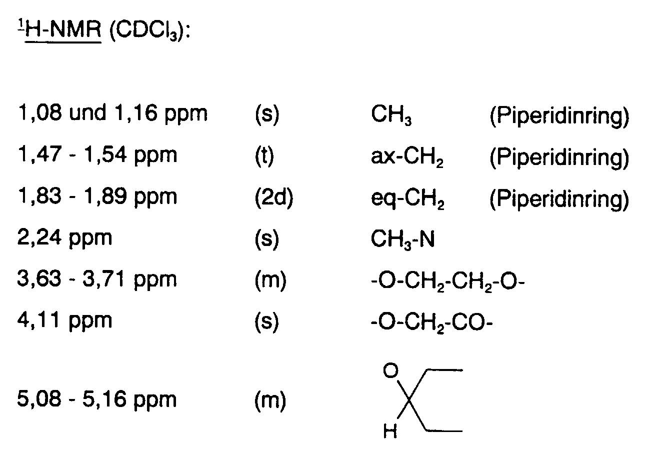

MALDI-MS: Mₙ = 955 M_{w} = 1005

### Beispiel 4: PEG-Disäure-bis-[di-(3,3,4,5,5-pentamethylpiperidinyl)amid]

### 4a) PEG-Disäuredichlorid

90 g (0,15 Mol) wasserfreie PEG-Disäure werden in 300 ml Chloroform gelöst und mit 150 g Thionylchlorid versetzt. Die Lösung wird am Rückfluss gekocht, bis keine SO₂-Entwicklung mehr feststellbar ist (nach ca. 2 Stunden). Man lässt die Reaktionslösung auf Raumtemperatur abkühlen und dampft anschliessend das überschüssige Thionylchlorid und das Lösungsmittel am Rotationsverdampfer ab. Den Rückstand trocknet man im Hochvakuum bei Raumtemperatur.
Man erhält 98 g (100 % d. Th.) einer Flüssigkeit.

| Mikroanalyse: | | |
|---|---|---|
| | berechnet | gefunden |
| C | 47,63 % | 48,03 % |
| H | 7,33 % | 7,12% |
| Cl | 10,84 % | 11,22 % |

### 4b) PEG-Disäure-bis-[di-(3,3,4,5,5-pentamethylpiperidinyl)amid]

In einem 750 ml Sulfierkolben, mit KPG-Rührer, Tropftrichter und Innenthermometer versehen, werden 30 g (46 mMol) des unter 4a) hergestellten PEG-Disäuredichlorids in 150 ml Chloroform unter Argon gelöst. In den Tropftrichter wird eine Lösung aus 30 g (92 mMol) Di-(3,3,4,5,5-pentamethylpiperidinyl)amin in 150 ml Chloroform vorgelegt. Das Amin wird bei ≤ 10°C langsam zugetropft. Anschliessend lässt man über Nacht abreagieren. Die klare Lösung giesst man auf 20 ml eiskalte, 50-%ige Natronlauge und schüttelt die organische Phase aus. Die organische Phase wird abgetrennt, mit Natriumsulfat getrocknet und das Lösungsmittel anschliessend abgedampft. Der Rückstand wird im Hochvakuum bei 50°C getrocknet.
Man erhält 102 g (90 % d. Th.) einer viskosen Flüssigkeit.

| Mikroanalyse: | | |
|---|---|---|
| | berechnet | gefunden |
| C | 64,49 % | 65,28 % |
| H | 10,42 % | 10,83% |
| N | 6,84 % | 7,72% |

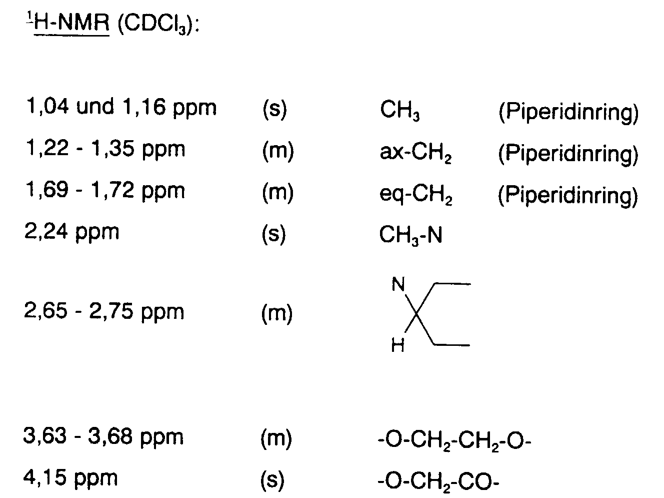

MALDI-MS: Mₙ = 1171 M_{w} = 1240

### Beispiel 5: PEG-Disäure-bis-di-(3,3,5,5-tetramethylpiperidinyl)amid]

In einem 1,5 Liter Sulfierkolben, mit KPG-Rührer, Innenthermometer und Tropftrichter versehen, werden 60 g (92 mMol) des gemäss Beispiel 4a) hergestellten PEG-Disäuredichlorids in 400 ml THF gelöst. Diese Lösung wird unter Argon auf 0°C abgekühlt. Im Tropftrichter wird eine Lösung von 54 g (195 mMol) Bis-(3,3,5,5-tetramethylpiperidinyl)amin in 200 ml THF vorgelegt. Diese Lösung tropft man langsam in den Reaktionskolben, so dass die Innentemperatur ≤ 10°C beträgt. Nach der vollständigen Zugabe wird über Nacht bei Raumtemperatur weitergerührt. Die dicke Suspension wird auf Eis und 15 g Natronlauge gegeben. Die erhaltene Lösung wird mit Kochsalz gesättigt und anschliessend mit 500 ml Chloroform extrahiert. Die organische Phase wird abgetrennt, mit Natriumsulfat getrocknet und anschliessend eingedampft. Der Rückstand wird im Hochvakuum getrocknet.
Man erhält 81 g (35 % d. Th.) einer viskosen Flüssigkeit.

| Mikroanalyse: | | |
|---|---|---|
| | berechnet | gefunden |
| C | 64,00 % | 63,96 % |
| H | 10,33 % | 10,31 % |
| N | 7,00 % | 7,87 % |

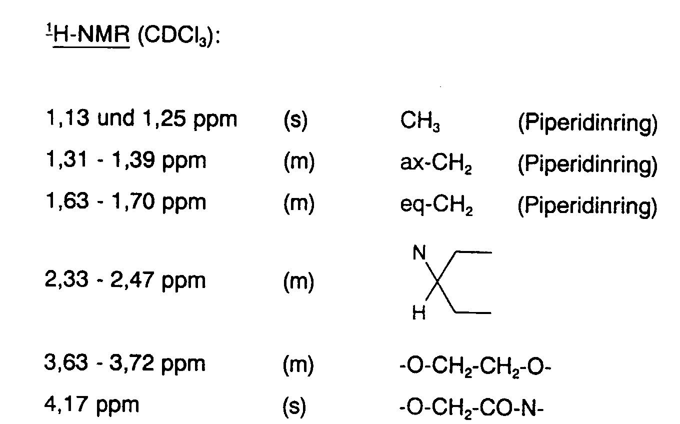

MALDI-MS: Mₙ = 1239 M_{w} = 1407

### Beispiel A1 : Lichtstabilisierung von Polypropylenfasern

2,5 g des erfindungsgemäßen Stabilisators aus Beispiel 1 werden zusammen mit 1 g Tris(2,4-di-tert.butylphenyl) phosphit, 1 g Calcium-Monoethyl-3,5-di-tert.butyl-4-hydroxybenzylphosphonat, 1 g Calciumstearat und 2,5 g TiO₂ (Kronos RN 57) in einem Turbomischer mit 1000 g Polypropylenpulver vermischt (Schmelzindex 12 g/10 min, gemessen bei 230°C/2,16 kg). Die Mischungen werden bei 200-230°C zu Granulat extrudiert; dieses wird anschließend mit Hilfe einer Pilotanlage (Leonard; Sumirago/VA, Italy) unter folgenden Bedingungen zu Fasern verarbeitet:
- Extrudertemperatur:: 190 - 230°C
- Kopftemperatur:: 255 - 260°C
- Streckverhältnis:: 1 : 3,5
- Strecktemperatur:: 100°C
- Fasern:: 10 den

Die so hergestellten Fasern werden vor weißem Hintergrund in einem Weather-O-Meter® Typ 65 WR (Atlas Corp.) mit einer Schwarztafeltemperatur von 63°C gemäß ASTM D 2565-85 belichtet. Nach unterschiedlichen Belichtungszeiten wird die verbliebene Zugfestigkeit der Proben gemessen. Aus den Meßwerten wird die Belichtungszeit T₅₀ berechnet, nach der die Zugfestigkeit der Proben nur noch halb so groß ist.

Zu Vergleichszwecken werden Fasern ohne erfindungsgemäßen Stablisator unter sonst gleichen Bedingungen hergestellt und getestet.

Die erfindungsgemäß stabilisierten Fasern weisen einen hervorragenden Festigkeitserhalt auf.

### Beispiel A2: Stabilisierung eines Zweischicht-Lackes

Der erfindungsgemässe Stabilisator aus Beispiel 1 wird in 5-10 g Xylol eingearbeitet und in einem Klarlack folgender Zusammensetzung geprüft:

| | |
|---|---|
| Synthacryl® SC 303 ¹⁾ | 27,51 g |
| Synthacryl® SC 370 ²⁾ | 23,34 g |
| Maprenal® MF 650 ³⁾ | 27,29 g |
| Butylacetat/Butanol (37/8) | 4,33 g |
| Isobutanol | 4,87 g |
| Solvesso® 150 ⁴⁾ | 2,72 g |
| Kristallöl K-30 ⁵⁾ | 8,74 g |
| Verlaufshilfsmittel Baysil® MA⁶⁾ | 1,20 g |
| | 100,00 g |

| | |
|---|---|
| 1) Acrylatharz, Fa. Hoechst AG; 65 % Lösung in Xylol/Butanol 26 : 9 | |
| 2) Acrylatharz, Fa. Hoechst AG; 75 % Lösung in Solvesso® 100 ⁴⁾ | |
| 3) Melaminharz, Fa. Hoechst AG; 55 % Lösung in Isobutanol | |
| 4) Hersteller: Fa. ESSO | |
| 5) Hersteller: Fa. Shell | |
| 6) 1 % in Solvesso® 150; Hersteller: Fa. Bayer AG | |

Dem Klarlack wird 1 % Stabilisator aus Beispiel 1 (in Xylol), bezogen auf den Feststoffgehalt des Lackes, zugesetzt. Als Vergleich dient ein Klarlack, der kein Lichtschutzmittel enthält.

Der Klarlack wird mit Solvesso® 100 auf Spritzfähigkeit verdünnt und auf ein vorbereitetes Aluminiumblech (coil coat, Füller, silbermetallic Basislack) gespritzt und bei 130°C 30 Minuten eingebrannt. Es resultiert eine Trockenfilmdicke von 40-50 µm Klarlack.

Die Proben werden dann in einem UVCON®-Bewitterungsgerät der Fa. Atlas Corp. (UVB-313 Lampen) bei einem Zyklus von 4 h UV-Bestrahlung bei 60°C und 4 h Kondensation bei 50°C bewittert.

Die Proben werden in regelmässigen Abständen auf Risse untersucht.

Die Proben mit dem erfindungsgemäßen Stabilisator weisen eine hohe Beständigkeit gegenüber Rissbildung auf.

### Beispiel A3: Stabilisierung eines photographischen Materials

0,087 g des Gelbkupplers der Formel 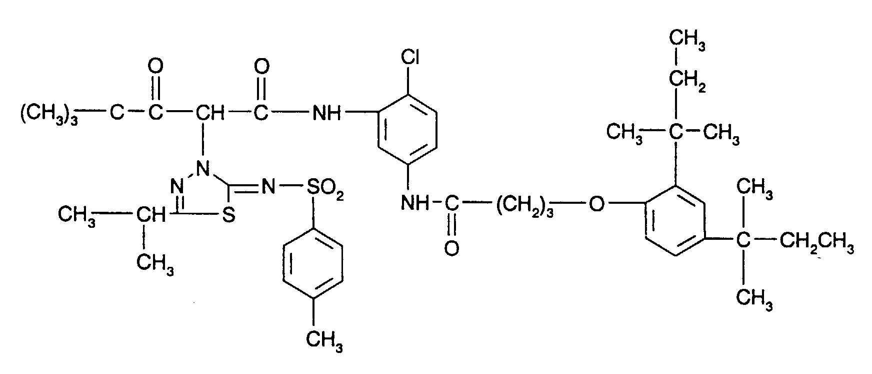 werden in 2,0 ml einer Lösung des erfindungsgemässen Stabilisators aus Beispiel 1 in Ethylacetat (2,25 g/100 ml) gelöst. Zu 1,0 ml dieser Lösung gibt man 9,0 ml einer 2,3 %-igen wässrigen Gelatinelösung, die auf einem pH-Wert von 6,5 eingestellt ist, und 1,744 g/l des Netzmittels der Formel 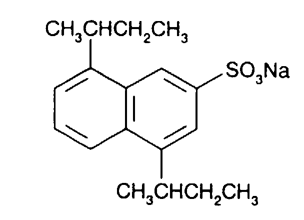 enthält.

Zu 5,0 ml der so erhaltenen Kuppleremulsion gibt man 2 ml einer Silberbromid-Emulsion mit einem Silbergehalt von 6,0 g/l sowie 1,0 ml einer 0,7 %-igen wässrigen Lösung des Härters der Formel 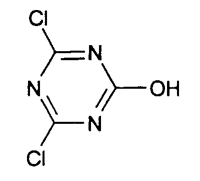 und vergiesst es auf ein 13 x 18 cm Kunststoff-beschichtetes Papier. Nach einer Härtungszeit von 7 Tagen werden die Proben hinter einem Silber-Stufenkeil mit 125 Lux·s belichtet und anschliessend im Ektaprint 2®-Prozess der Firma Kodak verarbeitet.

Die erhaltenen Gelbkeile werden in einem Atlas Weather-Ometer® mit einer 2500 W-Xenonlampe hinter einem UV-Filter (Kodak 2C) mit total 60 kJoule/cm² bestrahlt.

Eine Probe ohne Stabilisator läuft als Standard mit.

Der bei der Bestrahlung eingetretene Farbdichteverlust beim Absorptionsmaximum des gelben Farbstoffes wird mit einem Densitometer TR 924 A der Fa. Macbeth gemessen.

Der Lichtschutzeffekt ist aus dem Farbdichteverlust ersichtlich. Je kleiner der Dichteverlust, desto höher ist die Lichtschutzwirksamkeit.

Der erfindungsgemässe Stabilisator zeigt eine gute Lichtschutzwirkung.

### Beispiel A4: Stabilisierung von Polypropylen-Bändern

1,0 g des erfindungsgemäßen Stabilisators aus Beispiel 1 wird zusammen mit 1 g Tris(2,4-di-tert.butylphenyl)phosphit, 0,5 g Pentaerythrityl-tetrakis(3-[3',5'-di-tert.butyl-4'-hydroxyphenyl]--propionat) und 1 g Calciumstearat in einem Turbomischer mit 1000 g Polypropylenpulver vermischt (STATOILMF; Schmelzindex 4,0 g/10 min, gemessen bei 230°C/2,16 kg).

Die Mischungen werden bei 200-230°C zu Granulat extrudiert; dieses wird anschließend mit Hilfe einer Pilotanlage (Leonard; Sumirago/VA, Italy) unter folgenden Bedingungen zu 2,5 mm breiten Streckbändern von 50 µm Dicke verarbeitet:
- Extrudertemperatur:: 210 - 230°C
- Kopftemperatur:: 240 - 260°C
- Streckverhältnis:: 1 : 6
- Strecktemperatur:: 110°C

Die so hergestellten Bänder werden vor weißem Hintergrund in einem Weather-O-Meter® Typ 65 WR (Atlas Corp.) mit einer Schwarztafeltemperatur von 63°C gemäß ASTM D 2565-85 belichtet. Nach unterschiedlichen Belichtungszeiten wird die verbliebene Zugfestigkeit der Proben gemessen. Aus den Meßwerten wird die Belichtungszeit T₅₀ berechnet, nach der die Zugfestigkeit der Proben nur noch halb so groß ist.

Zu Vergleichszwecken werden Bänder ohne erfindungsgemäßen Stablisator unter sonst gleichen Bedingungen hergestellt und getestet.

Die erfindungsgemäß stabilisierte Probe weist einen hervorragenden Festigkeitserhalt auf.

### Beispiel A5: Stabilisierung von Polyamid 6

100 Teile unstabilisiertes Polyamid 6 Granulat (Ultramid® B3, Firma BASF) wird durch cryogene Mahlung pulverisiert und mit dem erfindungsgemässen Stabilisator aus Beispiel 1 versetzt. Die Mischung wird mit einem Henschel Mischer während 2 Minuten gemischt. Anschliessend wird das so erhaltene Pulver bei 80°C während 6 Stunden getrocknet und dann in einem Extruder (Typ Berstorff) bei maximal 240°C extrudiert und anschliessend granuliert. Das erhaltene Granulat wird auf einer Spritzgiessanlage bei maximal 240°C zu 1,0 mm dicken Hanteln und 2,0 mm dicken Platten verspritzt.

Die Hanteln werden in einem Umluftofen bei 140°C gealtert. Das Fortschreiten der Alterung wird durch Bestimmung der Spannungs-Dehnungs-Diagramme in Abständen von 1-3 Tagen verfolgt. Als Endpunkt wird die Zeit definiert, nach welcher die Restdehnung auf 50 % ihres Anfangwertes abgefallen ist. Je grösser die Zeit, desto besser ist die Stabilisierung.

Die Platten werden in einem Umluftofen bei 80°C während 1500 Stunden gealtert. Von diesen Platten wird der Yellowness Index (YI) nach ASTM D 1925-70 bestimmt. Niedrige Yl-Werte bedeuten wenig Verfärbung, hohe Yl-Werte starke Verfärbung der Muster. Je geringer die Verfärbung, desto wirksamer ist das Stabilisatorgemisch.

Zu Vergleichszwecken werden Proben ohne erfindungsgemässen Stablisator unter sonst gleichen Bedingungen hergestellt und getestet.

Der erfindungsgemässe Stabilisator zeigt eine gute Lichtschutzwirkung und die erfindungsgemäss stabilisierte Probe weist einen hervorragenden Festigkeitserhalt auf.

### Beispiel A6: Stabilisierung von Polyoxymethylen

Polyoxymethylen (Hostaform®C) wird in einem Brabender Plasticorder mit jeweils 0,3 Gew.-% Calciumstearat, 0,3 Gew.-% eines Stabilisators der Formel 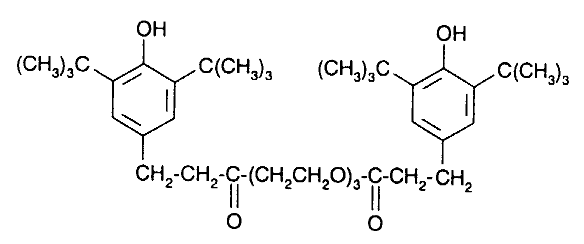 und 0,3 Gew.-% des erfindungsgemässen UV-Absorbers aus Beispiel 1 für 7 Minuten bei 190°C und 30 Umdrehungen/Minute geknetet.

Anschließend wird das Material bei 190°C unter einem Druck von 3000 psi zu Platten von 1 mm Dicke gepreßt; die Verarbeitungsdauer bei diesem Schritt beträgt 3 Minuten.

Die Platten werden bei 60°C und einer Luftfeuchtigkeit von 23 % mit einer UV-A Quelle im Abstand von 20 cm belichtet. Die UV-A Quelle besteht aus 5 Leuchtstofflampen TL/09 und 5 Lampen TL/12 (Wellenlängenbereich 295 - 400 nm). In regelmäßigen Intervallen wird der Yellowness Index (YI, Methode ASTM D-1925) gemessen, der bei UV-Belichtung von Polyoxymethylen ein Maximum durchläuft. Dieses Maximum wird verursacht durch ein erstes Auftreten von visuell noch nicht feststellbaren Mikrorissen. Bei weiterer Belichtung werden zu einem späteren Zeitpunkt Risse in den Platten sichtbar.

Zu Vergleichszwecken werden Proben ohne erfindungsgemässen Stabilisator unter sonst gleichen Bedingungen hergestellt und getestet.

Der erfindungsgemässe Stabilisator zeigt eine gute Lichtschutzwirkung, d. h. die Zeitdauer (in Wochen) bis zum Auftreten des Yl-Maximums bzw. der sichtbaren Rissbildung ist deutlich grösser als ohne erfindungsgemässen Stabilisator.

## Patentansprüche

1. Verbindungen der Formel 1 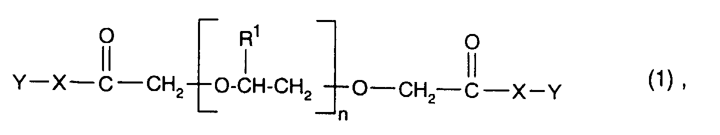 und Verbindungen der Formel 2 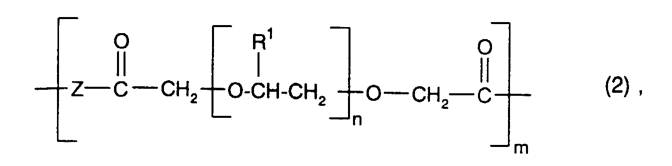 worin
R¹ H oder CH₃, und
Y die Gruppe 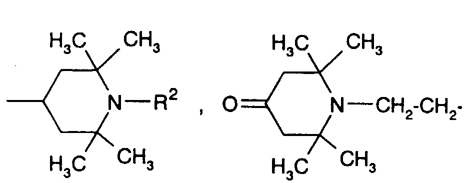 oder 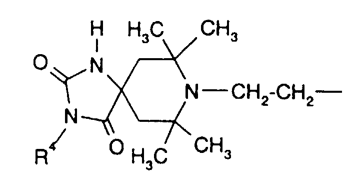 bedeutet,
X O oder NR³
R² H, C₁-C₂₀-Alkyl, C₁-C₂₀-Hydroxyalkyl, O-C₁-C₂₀-Alkyl, CO-C₁-C₂₀-Alkyl, O-C₅-C₈-Cydoalkyl, C₂-C₂₀-Alkenyl, C₂-C₂₀-Alkinyl, C₆-C₂₀-Aryl, CO-C₆-C₂₀-Aryl, C₇-C₂₀-Aralkyl, CO-C₇-C₂₀-Aralkyl, und
R³ H, C₁-C₂₀-Alkyl oder 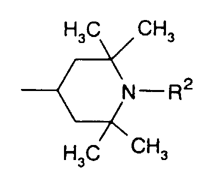 bedeuten, und
R⁴ H, C₁-C₂₀-Alkyl, CO-C₁-C₂₀-Alkyl, CO-C₆-C₂₀-Aryl oder CO-C₇-C₂₀-Aralkyl ist, und Z 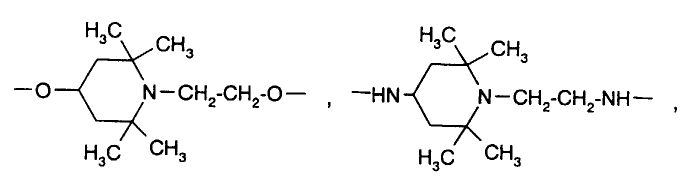 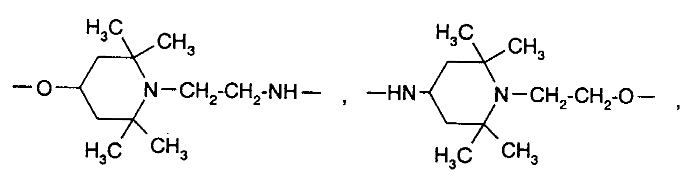 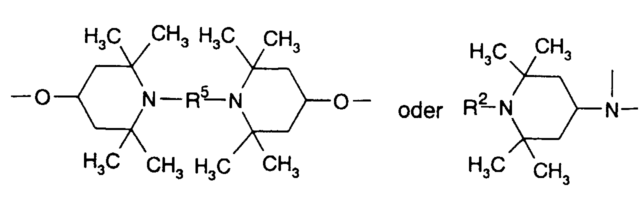 ist, und
R⁵ C₁-C₂₀-Alkylen, C₂-C₂₀-Alkenylen oder C₂-C₂₀-Alkinylen bedeuten, und
n eine Zahl von 1 bis 100 ist, und
m eine Zahl von 1 bis 100 ist.

2. Verbindungen gemäss Anspruch **1**, worin
R² H, C₁-C₁₂-Alkyl, C₁-C₁₂-Hydroxyalkyl, O-C₁-C₁₂-Alkyl, CO-C₁-C₁₂-Alkyl, O-C₅-C₈-Cycloalkyl, C₂-C₁₂-Alkenyl, C₂-C₁₂-Alkinyl, C₆-C₁₄-Aryl, CO-C₆-C₁₄-Aryl, C₇-C₁₅-Aralkyl oder CO-C₇-C₁₅-Aralkyl ist.

3. Verbindungen gemäss Anspruch **1**, worin
R² H, C₁-C₈-Alkyl, O-C₁-C₈-Alkyl, O-C₅-C₈-Cycloalkyl, C₂-C₈-Alkenyl oder C₂-C₈-Alkinyl ist.

4. Verbindungen gemäss Anspruch **1**, worin
R³ H, C₁-C₁₂-Alkyl oder 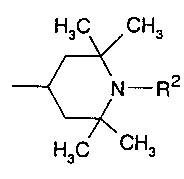 bedeuten, und
R⁴ H, C₁-C₁₂-Alkyl, CO-C₁-C₁₂-Alkyl, CO-C₆-C₁₄-Aryl oder CO-C₇-C₁₅-Aralkyl ist.

5. Verbindungen gemäss Anspruch **4**, worin
R³ H oder 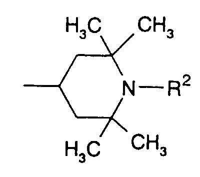 bedeutet, und
R⁴ H, C₁-C₈-Alkyl, CO-C₁-C₈-Alkyl, CO-Phenyl oder CO-Phenylalkyl ist.

6. Verbindungen gemäss Anspruch **1**, worin
Z 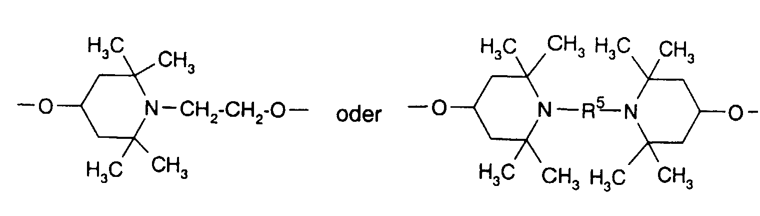 ist.

7. Verbindungen gemäss Anspruch **1**, worin
n eine Zahl von 1 bis 50 ist.

8. Verbindungen gemäss Anspruch **1**, worin
m eine Zahl von 1 bis 50 ist.

9. Verbindungen der Formel 1 gemäss Anspruch **1**.

10. Verbindungen gemäss Anspruch **9**, worin
Y eine Gruppe 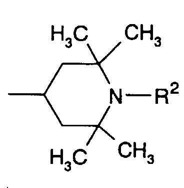 ist.

11. Verbindungen gemäss Anspruch **9**, worin
Y eine Gruppe 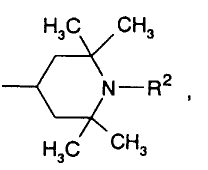 R² H oder C₁-C₄-Alkyl,
R³ H oder 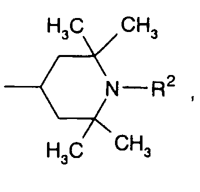 und
n eine Zahl von 5 bis 25 ist.

12. Zusammmensetzung enthaltend a) ein gegen Schädigung durch Licht, Sauerstoff und/oder Wärme empfindliches organisches Material und b) als Stabilisator mindestens eine Verbindung gemäss Anspruch **1**.

13. Zusammensetzung nach Anspruch **12**, worin die Komponente a) eine organisches Polymer ist.

14. Zusammensetzung nach Anspruch **12**, worin die Komponente a) ein synthetisches Polymer ist.

15. Zusammensetzung nach Anspruch **12**, worin die Komponente a) ein polares Polymer oder ein Lackbindemittel auf der Basis von Acryl-, Alkyd-, Polyurethan-, Polyester- oder Polyamidharz oder entsprechend modifizierter Harze ist.

16. Zusammensetzung nach Anspruch **12** enthaltend zusätzlich zu den Komponenten a) und b) weitere übliche Additive.

17. Zusammensetzung nach Anspruch **12** enthaltend 0,01 bis 10 Gew.-% der Komponente b) bezogen auf das Gewicht der Zusammensetzung.

18. Verfahren zur Stabilisierung von organischem Material gegen Schädigung durch Licht, Sauerstoff und/oder Wärme, dadurch gekennzeichnet, dass man diesem Material eine Verbindung gemäss Anspruch **1** beimischt.

19. Verwendung von Verbindungen gemäss Anspruch **1** zur Stabilisierung von organischem Material gegen Schädigung durch Licht, Sauerstoff und/oder Wärme.
